(19) Europäisches Patentamt
European Patent Office
Office européen des brevets

(11) **EP 4 632 537 A1**

(12) **EUROPEAN PATENT APPLICATION**
published in accordance with Art. 153(4) EPC

(43) Date of publication:
**15.10.2025 Bulletin 2025/42**

(21) Application number: **24832214.1**

(22) Date of filing: **29.04.2024**

(51) International Patent Classification (IPC):
**G06F 3/01** (2006.01)    **G06F 1/16** (2006.01)
**H04W 4/80** (2018.01)

(52) Cooperative Patent Classification (CPC):
**A61H 3/00; G16H 20/30; A61H 1/0244;
A61H 2003/007; A61H 2201/1652;
A61H 2201/5015; A61H 2201/5058**

(86) International application number:
**PCT/KR2024/005786**

(87) International publication number:
**WO 2025/005429 (02.01.2025 Gazette 2025/01)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.06.2023  KR 20230085183**

(71) Applicant: **Samsung Electronics Co., Ltd.
Suwon-si, Gyeonggi-do 16677 (KR)**

(72) Inventors:
• **AHN, Chiyoung
  Suwon-si, Gyeonggi-do 16677 (KR)**
• **KANG, Donghee
  Suwon-si, Gyeonggi-do 16677 (KR)**

(74) Representative: **Gulde & Partner
Patent- und Rechtsanwaltskanzlei mbB
Berliner Freiheit 2
10785 Berlin (DE)**

(54) **HAPTIC FEEDBACK CONTROL METHOD FOR CONTROLLING HAPTIC FEEDBACK OF WEARABLE DEVICE AND WEARABLE DEVICE AND ELECTRONIC DEVICE FOR PERFORMING SAME**

(57)    A haptic feedback control method for controlling haptic feedback of a wearable device and a wearable device and/or an electronic device for performing the same are disclosed. The haptic feedback control method may comprise the operations of: receiving sensor data including movement information of a user from a wearable device when an exercise program using the wearable device is executed; determining a threshold interval for haptic intensity of haptic feedback on the basis of at least one of user profile data of the user, exercise attribute data of the exercise program, and the sensor data; determining the haptic intensity of the haptic feedback to be provided to the user within the determined threshold interval for haptic intensity; and, when a haptic feedback delivery event occurs, transmitting, to the wearable device, a control signal for controlling the haptic feedback of the determined haptic intensity to be provided to the user.

Start

Start exercise program 810

Receive sensor data 820

Determine threshold interval of haptic intensity of haptic feedback 830

Determine haptic intensity of haptic feedback based on threshold interval of haptic intensity 840

Adjust threshold interval of haptic intensity 880

Has haptic feedback provision event occurred? 850

No

Yes

Transmit control signal to provide haptic feedback at determined haptic intensity to wearable device 860

Determine that user perceives haptic feedback? 870

No

Yes

End

**FIG. 8**

**Description**

1. Field

Certain example embodiments relate to a haptic feedback control technique for controlling haptic feedback of a wearable device, and/or a wearable device and an electronic device for performing the same.

2. Description of Related Art

Typically, a walking assistance device may be equipment or a device for assisting person to exercise, and/or for assisting a patient who is not able to walk by themselves due to various diseases or accidents to perform walking exercise for rehabilitation, and/or equipment or a device used for such exercise. As the number of aging individuals increases, a growing number of people experience inconvenience in walking or have difficulty walking normally due to malfunctioning joint issues, and there is increasing interest in walking assistance devices. A walking assistance device may be worn on a body of a user to assist the user with walking and/or exercise by providing the necessary muscular strength and/or to induce the user to walk in a normal walking pattern. The walking assistance device may perform a function to assist various leg exercises (e.g., power walking, jogging, stair climbing, lunge, stretching) of a user.

SUMMARY

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

In one general example aspect, a haptic feedback control method of controlling haptic feedback of a wearable device may include, when an exercise program is performed using the wearable device, receiving sensor data including motion information of a user from the wearable device, determining a threshold range with respect to haptic intensity of the haptic feedback based on at least one of user profile data of the user, exercise attribute data of the exercise program, and the sensor data, determining haptic intensity of haptic feedback to be provided to the user within the threshold range with respect to the determined haptic intensity, and when a haptic feedback provision event occurs, transmitting a control signal configured to control to provide the user with haptic feedback at the determined haptic intensity.

In another general example aspect, an electronic device may include a processor and a communication module, comprising communication circuitry, configured to communicate with a wearable device under control of the processor. At least one processor, comprising processing circuitry, may be individually and/or collectively configured to, when an exercise program is performed using the wearable device, receive sensor data including motion information of a user from the wearable device through the communication module and determine a threshold range with respect to haptic intensity of haptic feedback based on at least one of user profile data of the user, exercise attribute data of the exercise program, and the sensor data. The processor(s) may be configured to determine haptic intensity of haptic feedback to be provided to the user within the threshold range with respect to the determined haptic intensity. When a haptic feedback provision event occurs, the processor is configured to transmit a control signal configured to control to provide the user with haptic feedback at the determined haptic intensity through the communication module.

In another general example aspect, a wearable device may include a driving module, including a motor, configured to generate torque, a torque transmission frame configured to transmit the generated torque to a leg of a user, a thigh fastener connected, directly or indirectly, to the torque transmission frame and configured to connect the torque transmission frame to the leg of the user, a sensor module configured to obtain sensor data including motion information of the user when an exercise program using the wearable device is performed, a haptic module including a haptic actuator configured to provide haptic feedback to the user, a communication module, comprising communication circuitry, configured to receive haptic intensity data including information about haptic intensity of the haptic feedback from an electronic device, and a processor, comprising processing circuitry, configured to control haptic intensity generated by the haptic actuator based on information about the haptic intensity included in the haptic intensity data. A maximum and/or high intensity of the haptic intensity generated by the haptic actuator is included in a threshold range with respect to haptic intensity determined based on at least one of user profile data of the user, exercise attribute data of the exercise program, and the sensor data.

Other features and aspects will be apparent from the following detailed description, the drawings, and the claims.

BRIEF DESCRIPTION OF THE DRAWINGS

These and/or other aspects, features and advantages will become apparent and more readily understood from the following description of exemplary embodiments in conjunction with the accompanying drawings.

FIG. 1 is a diagram illustrating an overview of a wearable device worn on a body of a user according to an example embodiment.

FIG. 2 is a diagram illustrating an exercise assistance system including a wearable device and an electronic device according to an example embodiment.

FIG. 3 is a rear schematic diagram of a wearable device according to an example embodiment.

FIG. 4 is a left side view of a wearable device according to an example embodiment.

FIGS. 5A and 5B are diagrams illustrating a configuration of a control system of a wearable device, according to an example embodiment.

FIG. 6 is a diagram illustrating an interaction between a wearable device and an electronic device according to an example embodiment.

FIG. 7 is a diagram illustrating a configuration of an electronic device according to an example embodiment.

FIG. 8 is a flowchart illustrating operations of a haptic feedback control method of controlling haptic feedback of a wearable device according to an example embodiment.

FIG. 9 is a diagram illustrating a difference in perception of haptic intensity based on a user characteristic according to an example embodiment.

FIG. 10 is a diagram illustrating a difference in perception of haptic intensity based on exercise intensity of an exercise program according to an example embodiment.

FIG. 11 is a diagram illustrating the determination of a threshold range for haptic intensity of haptic feedback according to an example embodiment.

FIG. 12 is a diagram illustrating the determination of haptic intensity of haptic feedback in a threshold range for the haptic intensity according to an example embodiment.

FIG. 13 is a diagram illustrating a method of determining whether to perceive haptic feedback of a user according to an example embodiment.

FIG. 14 is a diagram illustrating adjustment of a threshold range for haptic intensity according to an example embodiment.

FIG. 15 is a diagram illustrating an example of adjusting haptic intensity based on sensor data according to an example embodiment.

FIGS. 16A to 16E are diagrams illustrating exercise situations in which a haptic feedback provision event occurs according to an example embodiment.

FIGS. 17A and 17B are diagrams illustrating applications using haptic feedback according to an example embodiment.

## DETAILED DESCRIPTION

The following detailed structural or functional description is provided as an example only and various alterations and modifications may be made to the examples. Accordingly, the embodiments are not construed as limited to the disclosure and should be understood to include all changes, equivalents, and replacements within the idea and the technical scope of the disclosure.

As used herein, the singular forms "a", "an", and "the" include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms, including technical and scientific terms, used herein have the same meaning as commonly understood by one of ordinary skill in the art to which this disclosure pertains. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

Hereinafter, certain example embodiments will be described in detail with reference to the accompanying drawings. When describing the examples with reference to the accompanying drawings, like reference numerals refer to like elements and a repeated description related thereto will be omitted.

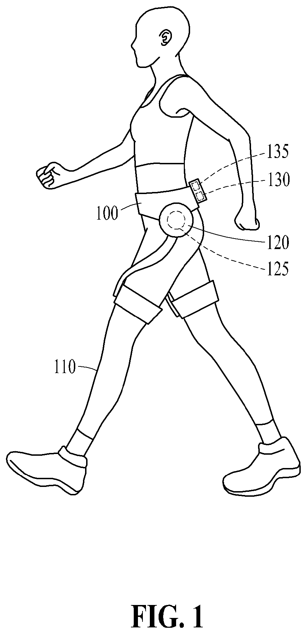

FIG. 1 is a diagram illustrating an overview of a wearable device worn on a user's body, according to an embodiment.

Referring to FIG. 1, in an embodiment, a wearable device 100 may be a device worn on a body of a user 110 to assist the user 110 in walking, exercising, and/or working. The wearable device 100 may be used to measure a physical ability (e.g., a walking ability, an exercise ability, or an exercise posture) of the user 110. In the embodiments, the term "wearable device" may be replaced with "wearable robot," "walking assistance device," or "exercise assistance device". The user 110 may be a human or an animal, but is not limited thereto. The wearable device 100 may be worn on the body (e.g., a lower body (legs, ankles, knees, etc.) or a waist) of the user 110 and may apply an external force, such as an assistance force and/or a resistance force, to a body motion of the user 110. The assistance force may be a force assisting the body motion of the user 110, which is applied in the same direction as a direction of the body motion of the user 110. The resistance force may be a force impeding the body motion of the user 110, which is applied in an opposite direction to the direction of the body motion of the user 110. The term "resistance force" may also be referred to as an "exercise load".

In an embodiment, the wearable device 100 may operate in a walking assistance mode for assisting the user 110 in walking. In the walking assistance mode, the wearable device 100 may assist the walking of the user 110 by applying an assistance force generated through a driving module 120 of the wearable device 100 to the body of the user 110. The wearable device 100 may expand a walking ability of the user 110 by allowing the user 110 to walk independently or walk for a long time by providing a force needed for the walking of the user 110. The wearable device 100 may also improve the walking of a user having an abnormal walking habit or posture.

In an embodiment, the wearable device 100 may operate in an exercise assistance mode for enhancing the exercise effect of the user 110. The exercise assistance mode may include a resistance mode and an assistance mode. The resistance mode of the exercise assistance mode may represent a mode for hindering a body motion of the user 110 or providing resistance to a body motion of the user 110 by applying a resistance force generated by the driving module 120 to a body of the user 110. When the wearable device 100 is a hip-type wearable device that is worn on the waist (or pelvis) and legs (e.g., thighs) of the user 110, in the resistance mode, the wearable device 100 may provide an exercise load to a leg motion of the user 110 while being worn on the legs, thereby enhancing the exercise effect on the legs of the user 110. The assistance mode of the exercise assistance mode may be a mode for applying an assistance force for assisting exercise of the user 110 to the body of the user 110. In the assistance mode, an assistance force in the same direction as a body motion may be provided to the user 110 to assist the body motion of the user. For example, when a person with a disability or an elderly person wears the wearable device 100 to exercise, the wearable device 100 may provide an assistance force to assist a body motion. In the assistance mode, the wearable device 100 may provide a force in the same direction as a direction of a leg motion of the user 110 and the user 110 may improve a walking speed and a walking distance with a small force. In an exercise program, the resistance mode and the assistance mode may be combined and operated. For example, the wearable device 100 may provide a combination of an assistance force and a resistance force for each exercise session or time interval in such a manner of providing an assistance force in one exercise session and providing a resistance force in another exercise session. In the exercise assistance mode, various exercise programs may be operated depending on the exercise purpose or a physical ability of the user 110. The exercise program may include, for example, cardio, strength training, posture balancing, or any combination thereof. The resistance mode and the assistance mode may be alternately activated appropriately depending on an exercise program performed by the wearable device 100 and a target exercise speed that is suitable to an appropriate physical condition (e.g., a heart rate) of the user 110 may be guided to the user during the exercise of the user 110. For example, the target exercise speed may be provided in the unit of kilometers per hour (km/h), but the example is not limited thereto.

In an embodiment, the wearable device 100 may operate in a physical ability measurement mode for measuring a physical ability of the user 110. The wearable device 100 may measure motion information of the user 110 using a sensor (e.g., an angle sensor 125 and an inertial measurement unit (IMU) 135) provided in the wearable device 100 during the walking and/or exercise of the user 110 and may assess the physical ability of the user 110 based on the measured motion information. For example, a gait index or an exercise ability indicator (e.g., muscular strength, endurance, balance, or exercise motion) of the user 110 may be estimated through the motion information of the user 110 measured by the wearable device 100. The physical ability measurement mode may include an exercise posture measurement mode for measuring an exercise posture (or an exercise motion) of the user 110.

In some embodiments, the description is provided based on an example in which the wearable device 100 is a hip-type wearable device as shown in FIG. 1. However, the embodiments are not limited thereto. As described above, the wearable device 100 may be worn on other body parts (e.g., upper arms, lower arms, hands, calves, or feet) other than the waist or legs (specifically, thighs). The shape and configuration of the wearable device 100 may vary depending on the body part on which the wearable device 100 is worn.

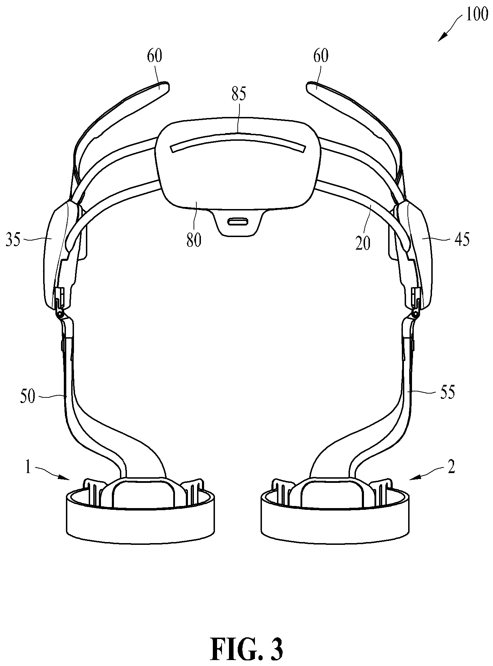

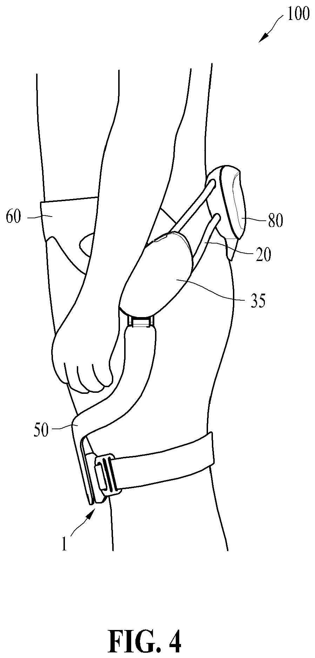

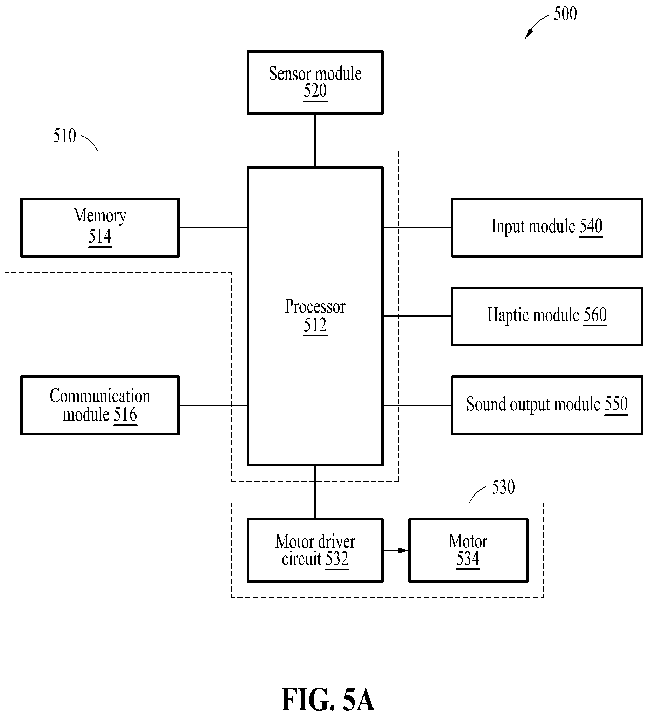

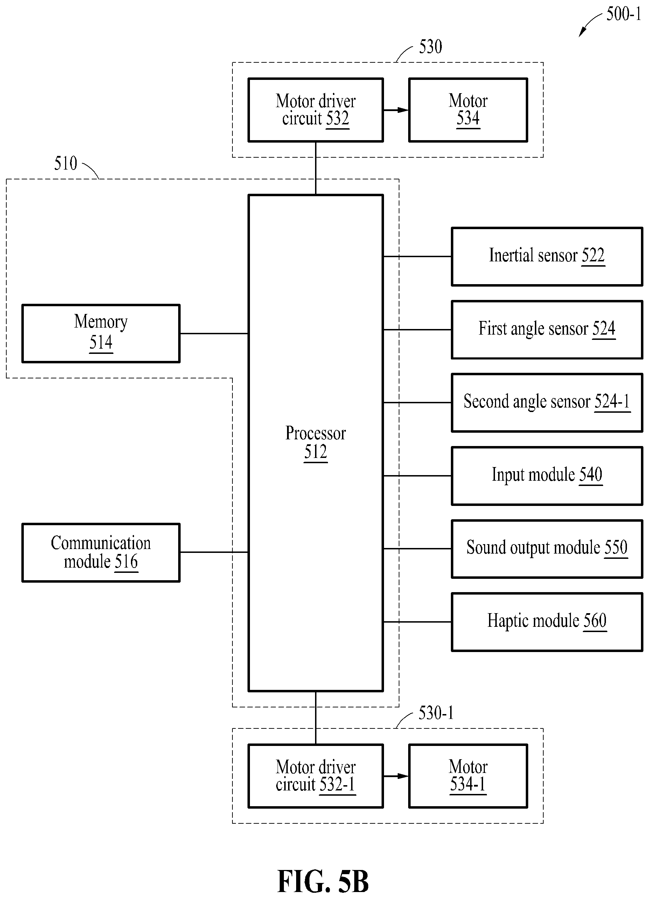

According to an embodiment, the wearable device 100 may include a support frame (e.g., a waist support frame 20 of FIGS. 3 and 4) for supporting the body of the user 110 when the wearable device 100 is worn on the body of the user 110, a driving module 120 (e.g., driving modules 35 and 45 of FIG. 3) configured to generate torque applied to legs of the user 110, a torque transmission frame (e.g., a first torque transmission frame 55 and a second torque transmission frame 50 of FIG. 3) configured to transmit torque generated by the driving module 120 to legs of the user 110, a sensor module (e.g., a sensor module 520 of FIG. 5A) including at least one sensor for obtaining sensor data including motion information about a body motion (e.g., a leg motion or an upper body motion) of the user 110, and a control module 130 (e.g., a control module 510 of FIGS. 5A and 5B) configured to control an operation of the wearable device 100. Each "driving module" herein may comprise a motor and/or circuitry.

The sensor module may include the angle sensor 125 and the IMU 135. The angle sensor 125 may measure a rotation angle of the torque transmission frame of the wearable device 100 corresponding to a hip joint angle value of the user 110. The rotation angle of the torque transmission frame measured by the angle sensor 125 may be estimated as a hip joint angle value (or a leg angle value) of the user 110. The angle sensor 125 may include, for example, an encoder and/or a hall sensor. In an embodiment, the angle sensor 125 may be disposed adjacent to a position where a motor included in the driving module 120 is connected, directly or indirectly, to the torque transmission frame. The IMU 135 may include an acceleration sensor and/or an angular velocity sensor, and may measure a change in acceleration and/or angular velocity according to a motion of the user 110. For example, the IMU 135 may measure a motion value of a waist support frame (e.g., the waist support frame 20 of FIG. 3) or a base body (e.g., a base body 80 of FIG. 3) of the wearable device 100. The motion value of the waist support frame or the base body measured by the IMU 135 may be estimated as a waist motion value or an upper body motion value of the user 110.

In an embodiment, the control module 130 and the IMU 135 may be disposed in the base body (e.g., the base body 80 of FIG. 3) of the wearable device 100. The base body may be on the waist of the user 110 when the user 110 wears the wearable device 100. The base body may be formed on or attached to the outside of the waist support frame of the wearable device 100.

In an embodiment, the wearable device 100 may provide haptic feedback to the user through a haptic module (e.g., a haptic module 560 of FIGS. 5A and 5B). The haptic module may include at least one haptic actuator configured to provide haptic feedback. The haptic feedback may be rapidly perceived by the user 110 without a separate confirmation procedure by the user 110 and may have an advantage over visual feedback and sound feedback because it is less limited by other factors, such as ambient noise. In haptic feedback, haptic intensity (or an intensity of the haptic feedback) may vary depending on a vibration intensity of the haptic actuator. Even when haptic feedback having the same haptic intensity occurs, a threshold intensity of the haptic feedback that the user 110 may feel and/or the haptic intensity felt by the user 110 may vary depending on a user characteristic (or user information) (e.g., age, gender, and physical information) of the user 110 and/or an exercise condition (e.g., a type of an exercise program, exercise intensity, and a user motion). It may be preferable to provide the user 110 with haptic feedback having an appropriate haptic intensity because when the haptic intensity is too low, the user 110 may not be able to perceive the haptic feedback and when the haptic intensity is too high, the user 110 may feel uncomfortable with the haptic feedback. In an embodiment, when providing haptic feedback during an exercise program using the wearable device 100, the wearable device 100 may provide haptic feedback having an appropriate haptic intensity, which is determined by considering a user characteristic and/or an exercise condition. The haptic intensity of the haptic feedback may be adjusted based on the user characteristic and/or the exercise condition in real-time. A further description of the determination of the appropriate haptic intensity of the haptic feedback is provided below.

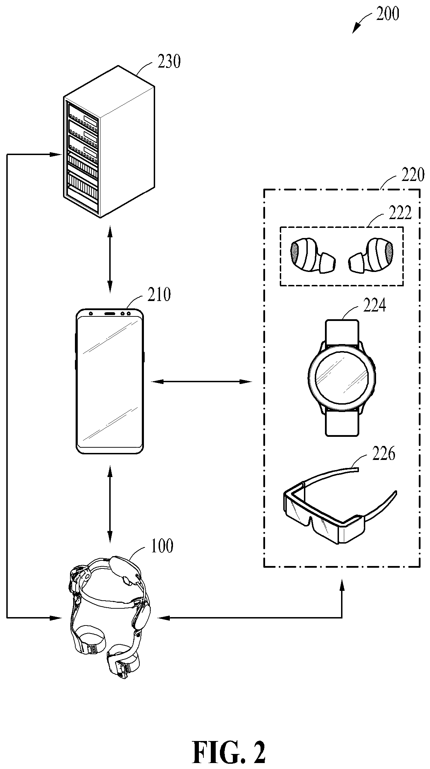

FIG. 2 is a diagram illustrating an exercise assistance system including a wearable device and an electronic device according to an embodiment.

Referring to FIG. 2, an exercise assistance system 200 may include the wearable device 100, an electronic device (or a user terminal) 210, another wearable device 220, and a server 230. In an embodiment, in the exercise assistance system 200, at least one of the devices described above (e.g., the another wearable device 220 or the server 230) or at least one device (e.g., a dedicated controller for the wearable device 100) may be added thereto.

In an embodiment, the wearable device 100 worn on a body of a user may assist the motion of the user in a walking assistance mode. For example, the wearable device 100 may be worn on the legs of the user to help the user in walking by generating an assistance force for assisting a leg motion of the user.

In an embodiment, the wearable device 100 may generate and apply a resistance force for hindering a body motion of the user and/or an assistance force for assisting a body motion of the user to the body of the user to enhance an effect of the user's exercise in the exercise assistance mode. In the exercise assistance mode, the user may select an exercise program (e.g., cardio such as power walking and outdoor walking, strength training such as squats, split lunges, dumbbell squats, and lunge and knee ups, a stretching exercise, a posture balancing exercise, or any combination thereof) that the user desires to conduct using the wearable device 100 through the electronic device 210 and/or exercise intensity applied to the exercise program. The wearable device 100 may control a driving module of the wearable device 100 based on the exercise program selected by the user and may obtain sensor data including motion information of the user through a sensor module. The wearable device 100 may adjust the strength of the resistance force and/or the assistance force applied to the user based on the exercise intensity selected by the user. For example, the wearable device 100 may control the driving module to generate a resistance force corresponding to the exercise intensity selected by the user. As the exercise intensity increases, the strength of the resistance force applied to the user may increase.

In an embodiment, the wearable device 100 may provide (or output) feedback (e.g., visual feedback, auditory feedback, or haptic feedback) corresponding to a state of the wearable device 100 in response to a control signal received from the electronic device 210. For example, the wearable device 100 may provide the visual feedback through a lighting unit (e.g., a lighting unit 85 of FIG. 3) and the auditory feedback through a sound output module (e.g., a sound output module 550 of FIGS. 5A and 5B).

The electronic device 210 may communicate with the wearable device 100, may remotely control the wearable device 100, or may provide the user with state information about a state (e.g., a booting state, a charging state, a sensing state, or an error state) of the wearable device 100. The electronic device 210 may recommend an exercise program using the wearable device 100 to the user and may analyze an exercise performed by the user. From the wearable device 100, the electronic device 210 may receive sensor data obtained by the sensor module of the wearable device 100 and may estimate a current exercise state, an exercise result, an exercise posture, and/or a physical ability of the user based on the received sensor data. The electronic device 210 may provide the user with the estimated exercise state, the exercise result, the exercise posture, and/or the physical ability of the user through a graphical user interface (GUI).

In an embodiment, the user may execute a program (e.g., an application) in the electronic device 210 to control the wearable device 100 and may adjust a torque magnitude output by a setting value (e.g., a driving module (e.g., the driving modules 35 and 45 of FIG. 3) or an operation of the wearable device 100, a sound volume of audio output from a sound output module (e.g., the sound output module 550 of FIGS. 5A and 5B), and a lighting unit (e.g., the brightness of the lighting unit 85 of FIG. 3)). The program executed by the electronic device 210 may provide a GUI for an interaction with the user. The electronic device 210 may be a device in various forms. For example, the electronic device 210 may include a portable communication device (e.g., a smartphone), a computer device, an access point, a portable multimedia device, or a home appliance (e.g., a television, an audio device, or a projector device), but examples are not limited to the foregoing devices.

In an embodiment, the electronic device 210 may be connected to the server 230 by using short-range wireless communication or cellular communication. The server 230 may receive user profile information of the user who uses the wearable device 100 from the electronic device 210 and may store and manage the received user profile information. The user profile information may include, for example, information about at least one of a name, an age, a gender, a height, a weight, medical history, or a body mass index (BMI). The server 230 may receive exercise history information about an exercise performed by the user from the electronic device 210 and may store and manage the received exercise history information. The server 230 may provide the electronic device 210 with various exercise programs or physical ability measurement programs to be provided to the user. In an embodiment, the server 230 may be connected to the wearable device 100. The server 230 may receive, from the wearable device 100, the sensor data measured by the wearable device 100 and may transmit, to the wearable device 100, a control signal for controlling an operation of the wearable device 100 and/or data related to the exercise program. In an embodiment, the server 230 may be a cloud server.

According to an embodiment, the wearable device 100 and/or the electronic device 210 may be connected, directly or indirectly, to the other wearable device 220. Exercise result information, physical ability information, and/or exercise posture assessment information of the user that are determined by the electronic device 210 may be transmitted to the other wearable device 220 and may be provided to the user through the other wearable device 220. The state information of the wearable device 100 may be transmitted to the other wearable device 220 and may be provided to the user through the other wearable device 220. In an embodiment, the wearable device 100, the electronic device 210, and the other wearable device 220 may be connected to each other via wireless communication (e.g., Bluetooth communication or wireless fidelity (Wi-Fi) communication). The other wearable device 220 may be, for example, wireless earphones 222, a smartwatch (or a watch-type wearable device) 224, or smartglasses (a wearable device in the type of glasses or goggles) 226, but is not limited to the devices described above.

In an embodiment, the wireless earphones 222 may be wirelessly connected to the electronic device 210 and/or the wearable device 100 and may output guiding voice, music, and/or a sound effect related to the exercise program.

In an embodiment, the smartwatch 224 may include a biometric sensor (e.g., a heart rate sensor and an electromyograph sensor) configured to measure a biosignal, including heart rate information of the user, and may transmit the biosignal measured by the biometric sensor to the electronic device 210 and/or the wearable device 100. For example, the electronic device 210 may estimate the heart rate information (e.g., a current heart rate, a maximum heart rate, and an average heart rate) and/or electromyography information of the user based on the biosignal received from the smartwatch 224, and may provide the user with the estimated heart rate information and/or the electromyography information. The heart rate information and/or the electromyography information may be used for the determination of the haptic intensity of the haptic feedback provided through the wearable device 100.

In an embodiment, the smartwatch 224 may include an inertial sensor configured to measure motion information of the user and/or a position sensor configured to measure position information of the user and may transmit the motion information and/or position information of the user to the electronic device 210 and/or the wearable device 100. The smartwatch 224 may include a communication module (e.g., a short-range communication module) for communicating with another device (e.g., the electronic device 210 and the wearable device 100). In an embodiment, the smartwatch 224 may provide an interface related to an exercise program through a display. The interface related to the exercise program may be implemented by a separate application installed in the smartwatch 224.

In an embodiment, the smartglasses 226 may provide information to the user through a display in the form of glasses. For example, the smartglasses 226 may output information about, for example, a current exercise speed, a target exercise speed, a currently achieved exercise amount, an exercise time, and biometric information, through a display in the exercise mode. In addition, the smartglasses 226 may output a screen for guiding an exercise path to the user.

In an embodiment, the wearable device 100 may provide the haptic feedback associated with an exercise posture of the user when the user performs exercise based on the exercise program. For example, when the exercise speed and/or the exercise posture satisfies a predetermined condition, the wearable device 100 may activate the haptic actuator and may provide the haptic feedback. The electronic device 210 may set and adjust the haptic intensity applied to at least one haptic actuator included in the wearable device 100. When a plurality of haptic actuators is provided, the same haptic intensity may be set to haptic actuators or the haptic intensity may be individually set to each haptic actuator. When the haptic intensity is individually set to each haptic actuator, the haptic intensities provided by the haptic actuators may differ from each other. The electronic device 210 may adjust the haptic intensity of the haptic feedback based on at least one of user profile data (e.g., a gender, an age, and physical information), exercise condition data (e.g., a type of an exercise program, exercise intensity, information about a user motion), and sensor data (e.g., heart rate information and electromyography information) received from the other wearable device 220. Since the wearable device 100 is a device worn on the user's body, it may be preferable to apply haptic stimulation at a natural haptic intensity that does not cause discomfort to the user when providing the haptic feedback. The electronic device 210 may decrease a possibility that the user may feel discomfort due to an excessive haptic intensity by appropriately adjusting the haptic intensity of the haptic feedback based on a characteristic and/or an exercise situation of the user.

FIG. 3 is a rear schematic diagram of a wearable device according to an embodiment. FIG. 4 is a left side view of a wearable device according to an embodiment.

Referring to FIGS. 3 and 4, the wearable device 100 in an embodiment may include a base body 80, a waist support frame 20, driving modules 35 and 45, torque transmission frames 50 and 55, thigh fasteners 1 and 2, and a waist fastener 60. The base body 80 may include a lighting unit 85. In an embodiment, at least one (e.g., the lighting unit 85) of the components described above may be omitted from the wearable device 100 or one or more other components may be added to the wearable device 100.

The base body 80 may be on the waist of a user when the user wears the wearable device 100. The base body 80 may be worn on the waist of the user to provide cushioning to the waist of the user and may support the waist of the user. The base body 80 may be hung on the hip part (an area of the hips) to prevent or reduce chances of the wearable device 100 from being downwardly separated due to gravity while the user wears the wearable device 100. The base body 80 may distribute a portion of the weight of the wearable device 100 to the waist of the user while the user wears the wearable device 100. The base body 80 may be connected, directly or indirectly, to the waist support frame 20. Waist support frame connecting elements (not shown) to be connected, directly or indirectly, to the waist support frame 20 may be provided at both ends of the base body 80.

In an embodiment, the lighting unit 85 may be provided on an outer surface of the base body 80. The lighting unit 85 may include a light source (e.g., a light-emitting diode (LED)). The lighting unit 85 may emit light in response to a control of a processor (not shown) (e.g., a processor 512 of FIGS. 5A and 5B) of the wearable device 100. In some embodiments, the lighting unit 85 may be controlled to provide (or output) visual feedback corresponding to the state of the wearable device 100 through the lighting unit 85.

The waist support frame 20 may support a body part (e.g., the waist) of the user when the wearable device 100 is worn on the body of the user. The waist support frame 20 may extend from both ends of the base body 80. The waist of the user may be accommodated inside the waist support frame 20. The waist support frame 20 may include at least one rigid body beam. Each beam may be in a curved shape having a preset curvature to enclose the waist of the user. The waist fastener 60 may be connected, directly or indirectly, to the end of the waist support frame 20. The driving modules 35 and 45 may be directly or indirectly connected to the waist support frame 20.

In an embodiment, a processor, a memory (e.g., a memory 514 of FIGS. 5A and 5B), an inertial sensor (e.g., the IMU 135 of FIG. 1 and an inertial sensor 522 of FIG. 5B), a communication module (e.g., a communication module 516 of FIGS. 5A and 5B), a sound output module (e.g., a sound output module 550 of FIGS. 5A and 5B), and a battery (not shown) may be disposed in the base body 80. The base body 80 may protect the components disposed therein. The processor may generate a control signal for controlling an operation of the wearable device 100. The processor may control a motor of the driving module 35 or 45. The processor and the memory may be included in a control circuit. The control circuit may further include a power supply circuit to provide power of the battery to each component of the wearable device 100.

In an embodiment, the wearable device 100 may include a sensor module (not shown) (e.g., the sensor module 520 of FIG. 5A) configured to obtain sensor data from at least one sensor. The sensor module may obtain sensor data including motion information of the user and/or motion information of a component of the wearable device 100. For example, the sensor module may include an inertial sensor (e.g., the IMU 135 of FIG. 1 and the inertial sensor 522 of FIG. 5B) configured to measure a motion value of the upper body of the user or a motion value of the waist support frame 20 and an angle sensor (e.g., the angle sensor 125 of FIG. 1, a first angle sensor 524 and a second angle sensor 524-1 of FIG. 5B) configured to measure a hip joint angle value of the user or a motion value of the torque transmission frame 50 or 55, but the example is not limited thereto. For example, the sensor module may further include at least one of a position sensor, a temperature sensor, a biosignal sensor, a distance sensor, or a proximity sensor.

The waist fastener 60 may be directly or indirectly connected to the waist support frame 20 and may fasten the waist support frame 20 to the waist of the user. The waist fastener 60 may include, for example, a pair of belts.

The driving module 35 or 45 may generate an external force (or torque) applied to the body of the user based on the control signal generated by the processor. For example, the driving module 35 or 45 may generate an assistance force or a resistance force applied to the legs of the user. In an embodiment, the driving modules 35 and 45 may include a first driving module 45 disposed at a position corresponding to a right hip joint of the user and a second driving module 35 disposed at a position corresponding to a left hip joint of the user. The first driving module 45 may include a first actuator and a first joint member and the second driving module 35 may include a second actuator and a second joint member. The first actuator may provide power transferred to the first joint member and the second actuator may provide power transferred to the second joint member. The first actuator and the second actuator may each include a motor configured to generate power (or torque) by receiving power from the battery. When the motor is driven as the power is supplied to the motor, the motor may generate a force (an assistance force) for assisting a body motion of the user or a force (a resistance force) for hindering a body motion of the user. In an embodiment, the control module may adjust the strength and direction of the force generated by the motor by adjusting a voltage and/or a current supplied to the motor.

In an embodiment, the first joint member and the second joint member may receive power from the first actuator and the second actuator, respectively, and may apply an external force to the body of the user based on the received power. In an embodiment, the first joint member and the second joint member may be disposed at positions corresponding to joints of the user, respectively. One side of the first joint member may be directly or indirectly connected to the first actuator and the other side of the first joint member may be directly or indirectly connected to the first torque transmission frame 55. The first joint member may be rotated by the power received from the first actuator. An encoder or a hall sensor that may operate as an angle sensor to measure a rotation angle (corresponding to a joint angle of the user) of the first joint member or the first torque transmission frame 55 may disposed on one side of the first joint member. One side of the second joint member may be connected to the second actuator, and the other side of the second joint member may be connected to the second torque transmission frame 50. The second joint member may be rotated by the power received from the second actuator. An encoder or a hall sensor that may operate as an angle sensor to measure a rotation angle of the second joint member or the second torque transmission frame 50 may be disposed on one side of the second joint member.

In an embodiment, the first actuator may be disposed in a lateral direction of the first joint member, and the second actuator may be disposed in a lateral direction of the second joint member. A rotation axis of the first actuator and a rotation axis of the first joint member may be spaced apart from each other, and a rotation axis of the second actuator and a rotation axis of the second joint member may also be spaced apart from each other. However, the embodiments are not limited thereto, and an actuator and a joint member may share a rotation axis. In an embodiment, each actuator may be spaced apart from a corresponding joint member. In this case, the driving module 35 or 45 may further include a power transmission module (not shown) configured to transmit power from the actuator to the joint member. The power transmission module may be a rotary body, such as a gear, or a longitudinal member, such as a wire, a cable, a string, a spring, a belt, or a chain. However, the scope of the embodiment is not limited by the positional relationship between an actuator and a joint member and the power transmission structure described above.

In an embodiment, when the wearable device 100 is worn on the user's legs, the first torque transmission frame 55 and the second torque transmission frame 50 may transmit torque generated by the first driving module 45 and the second driving module 35 to the body (e.g., legs) of the user, respectively. The transmitted torque may function as an external force applied to a leg motion of the user. Respective ends of the first torque transmission frame 55 and the second torque transmission frame 50 may be directly or indirectly connected to the joint member and may rotate. As the other ends of the first torque transmission frame 55 and the second torque transmission frame 50 are directly or indirectly connected to the first thigh fastener 2 and the second thigh fastener 1, the first torque transmission frame 55 and the second torque transmission frame 50 may transmit the torque generated by the first driving module 45 and the second driving module 35 to the user's thighs while supporting the user's thighs. For example, the first torque transmission frame 55 and the second torque transmission frame 50 may push or pull the user's thighs. The first torque transmission frame 55 and the second torque transmission frame 50 may extend in a longitudinal direction of the user's thighs or may be bent and enclose at least some portions of the circumferences of the user's thighs. The first torque transmission frame 55 may be a torque transmission frame for transmitting torque to the right leg of the user and the second torque transmission frame 50 may be a torque transmission frame for transmitting torque to the left leg of the user.

The first thigh fastener 2 and the second thigh fastener 1 may be directly or indirectly connected to the first torque transmission frame 55 and the second torque transmission frame 50, respectively, and may fasten the wearable device 100 to the legs (specifically, thighs) of the user. For example, the first thigh fastener 2 may be a thigh fastener for fastening the wearable device 100 to the right thigh of the user and the second thigh fastener 1 may be a thigh fastener for fastening the wearable device 100 to the left thigh of the user.

In an embodiment, the first thigh fastener 2 may include a first cover, a first fastening frame, and a first strap, and the second thigh fastener 1 may include a second cover, a second fastening frame, and a second strap. The first cover and the second cover may apply torques generated by the first driving module 45 and the second driving module 35 to the user's thighs, respectively. For example, the first cover and the second cover may be respectively disposed respective sides of the user's thighs and may push or pull the user's thighs. The first cover and the second cover may be disposed in the circumferential directions of the thighs of the user. The first cover and the second cover may extend to both sides from the other ends of the first torque transmission frame 55 and the second torque transmission frame 50 and may include curved surfaces corresponding to the thighs of the user. The respective ends of the first cover and the second cover may be directly or indirectly connected to the first fastening frame and the second fastening frame. The other ends of the first cover and the second cover may be directly or indirectly connected to the first strap and the second strap.

For example, the first fastening frame and the second fastening frame may be disposed to enclose at least some portions of the circumferences of the user's thighs, thereby the user's thighs may be prevented from being separated from the wearable device 100 or a possibility of separation may decrease. The first fastening frame may have a fastening structure that connects the first cover to the first strap, and the second fastening frame may have a fastening structure that connects the second cover to the second strap.

The first strap may enclose a remaining portion of the circumference of the right thigh of the user, which is not covered by the first cover and the first fastening frame, and the second strap may enclose a remaining portion of the circumference of the left thigh of the user, which is not covered by the second cover and the second fastening frame. The first strap and the second strap may include, for example, an elastic material (e.g., a band).

FIGS. 5A and 5B are diagrams illustrating a configuration of a control system of a wearable device, according to an embodiment.

Referring to FIG. 5A, a wearable device (e.g., the wearable device 100) may be controlled by a control system 500. The control system 500 may include a control module 510 (including a processing circuitry of a processor), a communication module 516 including a communication circuitry, a sensor module 520 including at least one sensor, a driving module 530 including a motor and/or a driver circuitry, an input module 540 including a circuitry, and a sound output module 550. The driving module 530 may include a motor 534 configured to generate power (e.g., torque) and a motor driver circuit 532 configured to drive the motor 534. Although FIG. 5A illustrates the driving module 530 including one motor driver circuit 532 and one motor 534, the example of FIG. 5A is merely an example. Referring to FIG. 5B, in a control system 500-1 as shown in the embodiment of FIG. 5B, a plurality (e.g., two or more) of motor driver circuits 532 and 532-1 and motors 534 and 534-1 may be provided. The driving module 530 including the motor driver circuit 532 and the motor 534 may correspond to the first driving module 45 of FIG. 3 and the driving module 530-1 including the motor driver circuit 532-1 and the motor 534-1 may correspond to the second driving module 35 of FIG. 3. The following descriptions of the motor driver circuit 532 and the motor 534 may also be respectively applicable to the motor driver circuit 532-1 and the motor 534-1 shown in FIG. 5B.

Referring back to FIG. 5A, the sensor module 520 may include at least one sensor configured to obtain sensor data. The sensor module 520 may transmit the obtained sensor data to the control module 510 (including a control circuit such as a processing circuit of a processor). The sensor module 520 may include a sensor configured to obtain sensor data including motion information of a user or motion information of a wearable device. The sensor module 520 may include an inertial sensor 522, a first angle sensor 524, and a second angle sensor 524-1 as shown in FIG. 5B. The inertial sensor 522 may measure an upper body motion value of the user. For example, the inertial sensor 522 may sense the acceleration and angular velocity of an X-axis, a Y-axis, and a Z-axis according to motion of the user. In addition, the inertial sensor 522 may obtain a motion value (e.g., an acceleration value and an angular velocity value) of a waist support frame of the wearable device. The first angle sensor 524 and the second angle sensor 524-1 may measure a hip joint angle value according to the leg motion of the user. The first angle sensor 524 may sense a change in the hip joint angle value of the right leg of the user and the second angle sensor 524-1 may sense a change in the hip joint angle value of the left leg of the user. For example, the first angle sensor 524 and the second angle sensor 524-1 may respectively include an encoder and/or a hall sensor. In addition, the first and second angle sensors 524 and 524-1 may obtain a motion value of a torque transmission frame of the wearable device. For example, the first angle sensor 524 may obtain a motion value (e.g., a rotation angle value) of the first torque transmission frame 55 and the second angle sensor 524-1 may obtain a motion value (e.g., a rotation angle value) of the second torque transmission frame 50.

In an embodiment, the sensor module 520 may further include a position sensor configured to obtain a position value of the wearable device, a proximity sensor configured to sense the proximity of an object, a biosignal sensor configured to detect a biosignal of a user, and a temperature sensor configured to measure an ambient temperature.

The input module 540 may receive a command or data to be used by another component (e.g., a processor 512) of the wearable device from the outside (e.g., the user) of the wearable device. The input module 540 may include, for example, a key (e.g., a button) or a touch screen.

The sound output module 550 may output a sound signal to the outside of the wearable device. The sound output module 550 may include a guide sound signal (e.g., a driving start sound or an operation error notification sound) and a speaker for playing music content or a guiding voice.

In an embodiment, the control system 500 may include a battery (not shown) to supply power to each component of the wearable device. The wearable device may convert the power of the battery into power suitable for an operating voltage of each component of the wearable device and supply the converted power to each component.

The driving module 530 may generate an external force to be applied to the user's legs by control of the control module 510. The driving module 530 may be in a position corresponding to a position of a hip joint of the user and may generate torque to be applied to the user's legs based on a control signal generated by the control module 510. The control module 510 may transmit the control signal to the motor driver circuit 532, and the motor driver circuit 532 may control an operation of the motor 534 by generating a current signal (or a voltage signal) corresponding to the control signal and supplying the current signal (or the voltage signal) to the motor 534. The current signal may not be supplied to the motor 534 according to the control signal. When the current signal is supplied to the motor 534, and the motor is driven, the motor 534 may generate an assistance force to assist the leg motion of the user or a resistance force to impede the leg motion of the user.

The control module 510 may control an overall operation of the wearable device, and may generate a control signal to control each component of the wearable device. The control module 510 may include a processor 512 and a memory 514.

The processor 512 may execute, for example, software to control at least one other component (e.g., a hardware or software component) of the wearable device directly or indirectly connected to the processor 512, and may perform a variety of data processing or computation. According to an embodiment, as at least a part of data processing or computation, the processor 512 may store instructions or data received from another component (e.g., the communication module 516) in the memory 514, may process the instructions or the data stored in the memory 514, and may store result data in the memory 514. According to an embodiment, the processor 512 may include a main processor (e.g., a central processing unit (CPU) or an application processor (AP)) or an auxiliary processor (e.g., a graphics processing unit (GPU), a neural processing unit (NPU), an image signal processor (ISP), a sensor hub processor, or a communication processor (CP)) that is operable independently of, or in conjunction with, the main processor. The auxiliary processor may be implemented separately from the main processor or as a part of the main processor.

Each "processor" herein includes processing circuitry, and/or may include multiple processors. For example, as used herein, including the claims, the term "processor" may include various processing circuitry, including at least one processor, wherein one or more of at least one processor, individually and/or collectively in a distributed manner, may be configured to perform various functions described herein. As used herein, when "a processor", "at least one processor", and "one or more processors" are described as being configured to perform numerous functions, these terms cover situations, for example and without limitation, in which one processor performs some of recited functions and another processor(s) performs other of recited functions, and also situations in which a single processor may perform all recited functions. Additionally, the at least one processor may include a combination of processors performing various of the recited /disclosed functions, e.g., in a distributed manner. At least one processor may execute program instructions to achieve or perform various functions.

The memory 514 may store a variety of data used by at least one component (e.g., the processor 512) of the control module 510. The variety of data may include, for example, software, sensor data, input data or output data for instructions related thereto. The memory 514 may include a volatile memory or a non-volatile memory (e.g., a random-access memory (RAM), a dynamic RAM (DRAM), or a static RAM (SRAM)).

The communication module 516 may support establishing a direct (e.g., wired) communication channel or a wireless communication channel between the control module 510 and another component of the wearable device or an external electronic device (e.g., the electronic device 210 or the other wearable device 220 of FIG. 2), and performing communication via the established communication channel. For example, the communication module 516 may transmit the sensor data obtained by the sensor module 520 to an external electronic device (e.g., the electronic device 210 of FIG. 2) and receive a control signal from the external electronic device. The communication module 516 may include one or more CPs that are operable independently of the processor 512 and that support a direct (e.g., wired) communication or a wireless communication. According to an embodiment, the communication module 516 may include a wireless communication module (e.g., a cellular communication module, a short-range wireless communication module, or a global navigation satellite system (GNSS) communication module), and/or a wired communication module. A corresponding one of these communication modules may communicate with another component of the wearable device and/or an external electronic device via a short-range communication network, such as Bluetooth™, wireless-fidelity (Wi-Fi), infrared data association (IrDA), or a long-range communication network, such as a legacy cellular network, a 5G network, a next-generation communication network, the Internet, or a computer network (e.g., a local area network (LAN) or a wide region network (WAN).

A haptic module 560 may include a haptic actuator configured to provide haptic feedback to the user. The haptic actuator may include, for example, a piezo actuator, a bander-type actuator, and/or a vibration motor-based actuator. One or a plurality of haptic actuators may be provided. In an embodiment, the haptic actuator may be disposed in at least one of a base body (e.g., the base body 80 of FIG. 3), a torque transmission frame (e.g., the first torque transmission frame 55 and the second torque transmission frame 50 of FIG. 3), and a thigh fastener (e.g., the first thigh fastener 2 and the second thigh fastener 1 of FIG. 3) of the wearable apparatus 100. In an embodiment, when the haptic module 560 include a plurality of haptic actuators, haptic intensities of haptic feedback provided by the haptic actuators may be different from each other.

The wearable device 100 in an embodiment may include the driving modules 530 and 530-1 including motors 534 and 534-1 configured to generate torque, a torque transmission frame (e.g., the first torque transmission frame 55 and the second torque transmission frame 50 of FIG. 3) configured to transmit the generated torque to the user's legs, a thigh fastener (e.g., the first thigh fastener 2 and the second thigh fastener 1 of FIG. 3) connected to the torque transmission frame and configured to connect the torque transmission frame to the user's legs, the sensor module 520 configured to obtain sensor data including motion information of the user when an exercise program is performed using the wearable device 100, the haptic module 560 including a haptic actuator configured to provide haptic feedback to the user, the communication module 516 configured to receive haptic intensity data including information about the haptic intensity of haptic feedback from the electronic device 210, and the processor 512 configured to control the haptic intensity generated by the haptic actuator based on the information about the haptic intensity included in the haptic intensity data. The wearable device 100 may further include a base body (e.g., the base body 80 of FIG. 3) configured to support the waist of the user.

A maximum intensity of the haptic intensity generated by the haptic actuator may be included in a threshold range for the haptic intensity determined based on at least one of user profile data of the user, exercise attribute data of an exercise program, and sensor data. A detailed description of the determination of the threshold range of the haptic intensity is provided below. The threshold range of the haptic intensity may be determined by the electronic device 210, the wearable device 100, and/or a server (e.g., the server 230 of FIG. 2).

"Based on" as used herein covers based at least on.

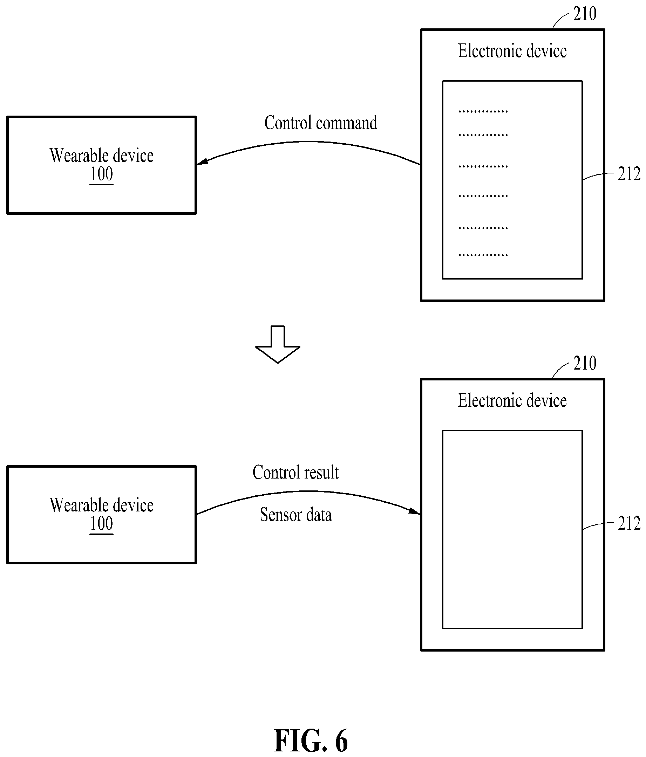

FIG. 6 is a diagram illustrating an interaction between a wearable device and an electronic device according to an embodiment.

Referring to FIG. 6, the wearable device 100 may communicate with the electronic device 210. For example, the electronic device 210 may be a user terminal of a user of the wearable device 100. In an embodiment, the wearable device 100 and the electronic device 210 may be connected to each other via short-range wireless communication (e.g., Bluetooth™ or Wi-Fi communication).

In an embodiment, the electronic device 210 may check a state of the wearable device 100 or execute an application to control or operate the wearable device 100. A screen of a user interface (UI) may be displayed to control an operation of the wearable device 100 or determine an operation mode of the wearable device 100 on a display 212 of the electronic device 210 through the execution of the application. The UI may be, for example, a graphical user interface (GUI).

In an embodiment, the user may input an instruction for controlling the operation of the wearable device 100 (e.g., an execution instruction to a walking assistance mode or an exercise assistance mode) or may change settings of the wearable device 100 through a GUI screen on the display 212 of the electronic device 210. The electronic device 210 may generate a control instruction (or control signal) corresponding to an operation control instruction or a setting change instruction input by the user and transmit the generated control instruction to the wearable device 100. The wearable device 100 may operate according to the received control instruction and transmit a control result according to the control instruction and/or sensor data measured by the sensor module of the wearable device 100 to the electronic device 210. The electronic device 210 may provide the user with result information (e.g., current exercise status information, exercise result information, exercise posture assessment information, and physical ability assessment information) derived by analyzing the control result and/or sensor data through the GUI screen.

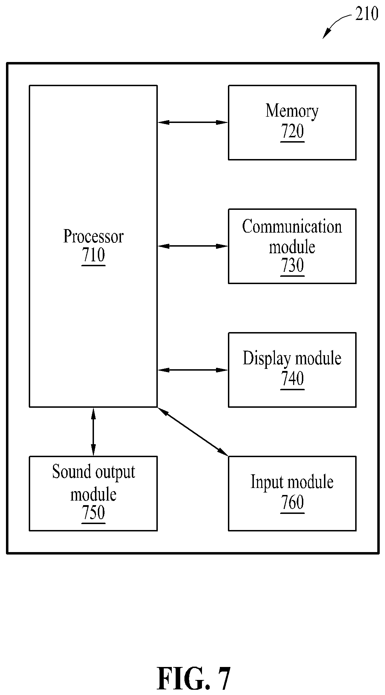

FIG. 7 is a diagram illustrating a configuration of an electronic device according to an embodiment.

Referring to FIG. 7, the electronic device 210 may include a processor 710, a memory 720, a communication module 730, a display module 740, a sound output module 750, and an input module 760. In an embodiment, at least one of the components (e.g., the sound output module 750) may be omitted from the electronic device 210, or one or more other components (e.g., a sensor module, a haptic module, and a battery) may be added to the electronic device 210.

The processor 710 may control at least one other component (e.g., a hardware or software component) of the electronic device 210 and may perform various types of data processing or operations. In an embodiment, as at least a part of data processing or operations, the processor 710 may store instructions or data received from another component (e.g., the communication module 730) in the memory 720, process the instructions or the data stored in the memory 720, and store result data in the memory 720.

In an embodiment, the processor 710 may include a main processor (e.g., a CPU or an AP) or an auxiliary processor (e.g., a GPU, an NPU, an ISP, a sensor hub processor, or a CP) that is operable independently of or in conjunction with the main processor.

The memory 720 may store various pieces of data used by at least one component (e.g., the processor 710 or the communication module 730) of the electronic device 210. The data may include, for example, a program (e.g., an application), and input data or output data for a command related thereto. The memory 720 may include at least one instruction executable by the processor 710. The memory 720 may include, for example, a volatile memory or a non-volatile memory. In this case, each processor may include a processing circuit.

The communication module 730 may support establishing a direct (e.g., wired) communication channel or a wireless communication channel between the electronic device 210 and another electronic device (e.g., the wearable device 100, the other wearable devices 220, and the server 230), and performing communication via the established communication channel. The communication module 730 may include a communication circuit for performing a communication function. The communication module 730 may include one or more CPs that are operable independently of the processor 710 (e.g., an AP) and that support a direct (e.g., wired) communication or a wireless communication. In an embodiment, the communication module 730 may include a wireless communication module (e.g., a Bluetooth™ communication module, a cellular communication module, a Wi-Fi communication module, or a GNSS communication module) that performs wireless communication or a wired communication module (e.g., a LAN communication module or a power line communication (PLC) module). For example, the communication module 730 may transmit a control instruction to the wearable device 100 and receive, from the wearable device 100, at least one of sensor data including body motion information of the user who is wearing the wearable device 100, state data of the wearable device 100, or control result data corresponding to the control instruction.

The display module 740 may visually provide information to the outside (e.g., the user) of the electronic device 210. The display module 740 may include, for example, a light-emitting diode (LCD) or organic light-emitting diode (OLED) display, a hologram device, or a projector device. The display module 740 may further include a control circuit to control the driving of the display. In an embodiment, the display module 740 may further include a touch sensor set to sense a touch or a pressure sensor set to sense the intensity of a force generated by the touch. The display module 740 may output a UI screen to control the wearable device 100 or provide various pieces of information (e.g., exercise evaluation information or setting information of the wearable device 100).

The sound output module 750 may output a sound signal to the outside of the electronic device 210. The sound output module 750 may include a speaker configured to play back a guiding sound signal (e.g., an operation start sound or an operation error alarm), music content, or a guiding voice based on the state of the wearable device 100. For example, when it is determined that the wearable device is not normally worn on the body of the user, the sound output module 750 may output a guiding voice for notifying the user of abnormal wearing of the wearable device 100 or guiding the user to wear the wearable device 100 normally.

The input module 760 may receive a command or data to be used by a component (e.g., the processor 710) of the electronic device 210, from the outside (e.g., the user) of the electronic device 210. The input module 760 may include an input component circuit and receive a user input. The input module 760 may include, for example, a touch recognition circuit for recognizing a touch on a key (e.g., a button) and/or a screen.

The electronic device 210 in an embodiment may include the processor 710 and the communication module 730 configured to communicate with the wearable device 100 under the control of the processor 710. The processor 710 may receive sensor data including motion information of a user from the wearable device 100 through the communication module 730, when an exercise program using the wearable device 100 is performed. The processor 710 may determine a threshold range with respect to the haptic intensity of haptic feedback based on at least one of user profile data of the user, exercise attribute data of the exercise program, and the sensor data received from the wearable device 100.

In an embodiment, the processor 710 may determine a first boundary value of the threshold range based on at least one of the user profile data of the user, the exercise attribute data of the exercise program, and the sensor data and may determine a second boundary value of the threshold range by applying a weight to the determined first boundary value. The threshold range of the haptic intensity may correspond to an interval between the first boundary value and the second boundary value. When the first boundary value is a lower limit of the threshold range, the second boundary value may be an upper limit of the threshold range, and when the first boundary value is the upper limit of the threshold range, the second boundary value may be the lower limit of the threshold range.

In an embodiment, when at least one of the exercise attribute data and the sensor data has changed while the exercise program is performed, the processor 710 may adjust the determined threshold range of the haptic intensity. For example, when the exercise program and/or the exercise intensity changes while the exercise program is performed, the processor 710 may determine the threshold range of the haptic intensity again based on the changed exercise program and/or the exercise intensity. When the motion information of the user measured by the wearable device 100 has significantly changed while the exercise program is performed, the processor 710 may determine the threshold range of the haptic intensity again based on the changed motion information of the user.

In an embodiment, the processor 710 may determine the haptic intensity of haptic feedback to be provided to the user within the threshold range of the determined haptic intensity. The processor 710 may determine the haptic intensity value to be provided to the user from haptic intensity values, which are greater than or equal to the lower limit and less than or equal to the upper limit of the threshold range. For example, the processor 710 may extract a plurality of different haptic intensity values in the threshold range of the haptic intensity and may determine one of the extracted haptic intensity values to be the haptic intensity value of the haptic feedback to be provided to the user. For example, a smallest value, a median value, or a largest value of the haptic intensity values may be determined to be the haptic intensity value of the haptic feedback to be provided to the user.

In an embodiment, when a haptic feedback provision event occurs, the processor 710 may transmit a control signal to provide the user with the haptic feedback at the determined haptic intensity to the wearable device 100 through the communication module 730. For example, in at least one of a case in which a hip joint angle according to the exercise posture of the user reaches a reference angle and a case in which the exercise posture of the user deviates a reference posture, the haptic feedback provision event may occur. In addition, when notifying the user of an increase or a decrease in the exercise intensity during the exercise, when requesting the user for an increase or a decrease in the exercise speed, and/or when sending an alert to the user as a heart rate of the user reaches a reference value, it may be determined that the haptic feedback provision event has occurred.

In an embodiment, the processor 710 may determine a haptic pattern based on a type of the haptic feedback provision event. For example, when an exercise program of walking exercise is performed and the target exercise speed of the walking exercise increases, the processor 710 may generate a control signal to decrease a cycle of a haptic pattern of the haptic feedback provided through the wearable device 100. Based on the threshold range of the determined haptic intensity, the processor 710 may determine the haptic intensity (or a maximum intensity of the haptic intensity of the haptic pattern) of the determined haptic pattern.

In an embodiment, after the haptic feedback is provided to the user, the processor 710 may determine whether the user has perceived the haptic feedback. For example, whether the user has perceived the haptic feedback may be determined based on whether the user performed an operation corresponding to the haptic feedback after the haptic feedback is provided. Alternatively, whether the user has perceived the haptic feedback may be determined based on perception assessment information on the haptic feedback input by the user through the input module 760. After providing the haptic feedback, the electronic device 210 may ask the user whether the user has perceived the haptic feedback through an application and the user may input the perception assessment information on the haptic feedback through the input module 760. The perception assessment information may include, for example, perception of the haptic feedback and/or the intensity of the haptic feedback.

In response to the determination that the user fails to perceive the haptic feedback, the processor 710 may adjust the threshold range of the haptic intensity. For example, the processor 710 may increase the lower limit of the threshold range of the haptic intensity. As the lower limit increases, the upper limit of the threshold range of the haptic intensity may increase or may be maintained without a change.

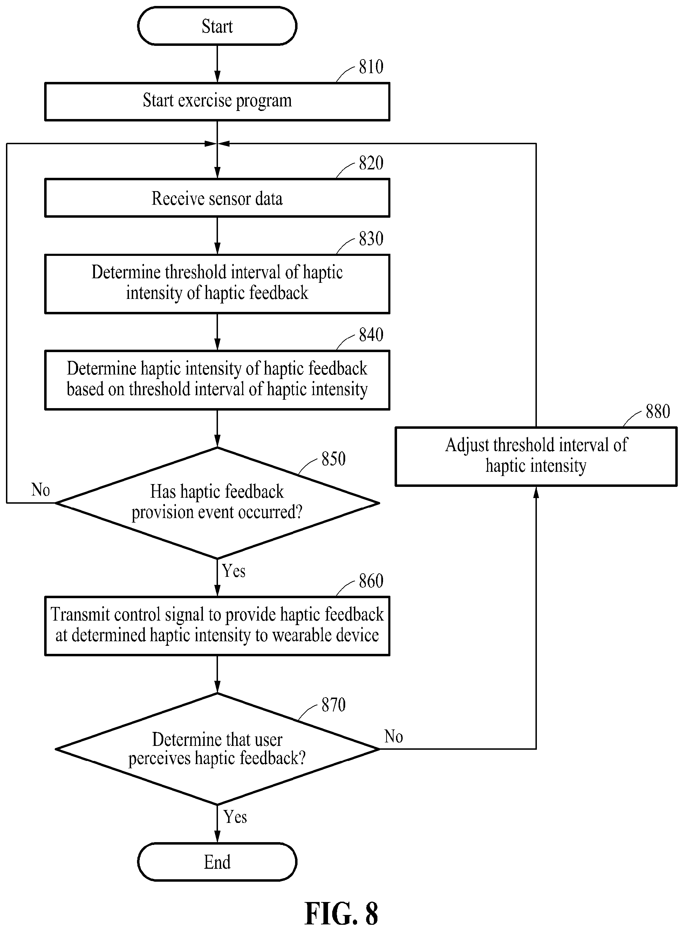

FIG. 8 is a flowchart illustrating operations of a haptic feedback control method of controlling haptic feedback of a wearable device according to an embodiment. In an embodiment, at least one of the operations of FIG. 8 may be simultaneously or parallelly performed with one another, and the order of the operations may be changed. In addition, at least one of the operations may be omitted, or another operation may be additionally performed.

Referring to FIG. 8, in operation 810, an exercise program may begin. For example, the electronic device 210 may recommend multiple exercise programs to a user and an exercise program selected by the user from the multiple exercise programs may begin. The electronic device 210 may select recommended exercise programs to be recommended to the user from all exercise programs based on user information (e.g., gender, age, physical condition, and medical history), the purpose of the exercise (e.g., losing a weight, managing a body shape, increasing strength, increasing endurance, preventing or reducing chances of a chronic disease, and increasing stamina) and/or the exercise history. Upon initiation of the exercise program, the user may perform an exercise process according to the exercise program while wearing the wearable device 100.

In an embodiment, immediately after the exercise program is selected or at the beginning of the exercise program, the threshold range of the haptic intensity may be determined based on the user profile data including the user information (e.g., a gender, an age, and a physical condition) and/or exercise attribute data (e.g., a type of the exercise program and a set exercise intensity) of the exercise program and the haptic intensity of the haptic feedback to be provided through the wearable device 100 may be determined within the determined threshold range. Alternatively, immediately after the exercise program is selected or at the beginning of the exercise program, a default haptic intensity that is preset may be applied.

Thereafter, the electronic device 210 may determine an appropriate haptic intensity for the haptic feedback by considering a user characteristic and/or an exercise condition according to the following operations and may control the wearable device 100 to provide the user with the haptic feedback at the determined appropriate haptic intensity.

In operation 820, the electronic device 210 may receive sensor data including motion information of the user from the wearable device 100 when the exercise program is performed using the wearable device 100. The electronic device 210 may receive the sensor data from not only the wearable device 100 but also another device (e.g., the smartwatch 224 of FIG. 2). For example, the electronic device may receive the sensor data including biometric information (e.g., heart rate information and electromyography information) of the user from the smartwatch.

In operation 830, the electronic device 210 may determine a threshold range of the haptic intensity of the haptic feedback. The threshold range may be an intensity interval that a haptic intensity may have (or may be set to) of haptic feedback provided by the wearable device 100.

In an embodiment, the electronic device 210 may determine the threshold range of the haptic intensity of the haptic feedback based on at least one of user profile data of the user, exercise attribute data of the exercise program, and the sensor data. The user profile data may include, for example, information about at least one of an age of the user and a gender of the user. The user profile data may further include at least one of information on physical information of the user (e.g., a height and a weight) and information on the medical history. The exercise attribute data may include, for example, a type of the exercise program and exercise intensity set to the exercise program. The sensor data may include, for example, information about at least one of acceleration according to a motion of the user, velocity according to the motion of the user, a hip joint angle of the user, and a heart rate of the user. The information about the acceleration and velocity according to the motion of the user may be obtained by an inertial sensor (e.g., the inertial sensor 522 of FIG. 5B) of the wearable device 100 and the information about the hip joint angle of the user may be obtained by an angle sensor (e.g., the first angle sensor 524 and the second angle sensor 524-1 of FIG. 5B) of the wearable device 100. The information about the heart rate of the user may be obtained by a heart rate sensor of the smartwatch (e.g., the smartwatch 224 of FIG. 2). The electronic device 210 may score the information of the sensor data and may determine the threshold range of the haptic intensity based on a scored result.

According to an embodiment, the operation of determining the threshold range of the haptic intensity may include an operation of determining the lower limit of the threshold range of the haptic intensity to be high as the exercise intensity of the exercise program increases.

According to an embodiment, the operation of determining the threshold range of the haptic intensity may include an operation of determining the lower limit of the threshold range of the haptic intensity to be high as the user is old.

According to an embodiment, the lower limit of the threshold range of the haptic intensity that is set when the user is male may be greater than the lower limit of the threshold range of the haptic intensity that is set when the user is female.

According to an embodiment, an operation of determining the threshold range of the haptic intensity may perform an operation of adjusting the threshold range of the determined haptic intensity when at least one of the exercise attribute data and the sensor data is changed during the exercise program. Based on the changed exercise attribute data and/or the changed sensor data, the threshold range of the haptic intensity may be determined again.

According to an embodiment, the operation of determining the threshold range of the haptic intensity may include an operation of determining a first boundary value of the threshold range based on at least one of the user profile data of the user, the exercise attribute data of the exercise program, and the sensor data and an operation of determining a second boundary value of the threshold range by applying a weight to the determined first boundary value. In this case, the threshold range of the haptic intensity may correspond to an interval between the first boundary value and the second boundary value. When the first boundary value is the lower limit of the threshold range, the second boundary value may be the upper limit of the threshold range. When the first boundary value is the upper limit of the threshold range, the second boundary value may be the lower limit of the threshold range.

In an embodiment, the electronic device 210 may determine the first boundary value and the second boundary value of the threshold range of the haptic intensity using a haptic intensity determination model. The haptic intensity determination model may be, for example, a mathematical model configured to determine the first boundary value to be a weighted sum of feature values included in at least one of the user profile data of the user, the exercise attribute data, and the sensor data and determine the second boundary value to be a value obtained by adding the first boundary value to a result value of applying a weight to the first boundary value. For example, a mathematical model for determining the first boundary value in the haptic intensity determination model may be the same as Equation 1 shown below.

[Equation 1]

$$X_L = w_0 + w_{11} \times U_1 + w_{12} \times U_2 + \ldots + w_{21} \times P_1 + w_{22} \times P_2 + \ldots + w_{31} \times S_1 + w_{32} \times S2 + \ldots$$

In this case, $X_L$ may denote the first boundary value (assuming that the first boundary value is the lower limit) of the threshold range of the haptic intensity and $W_0$ may denote a weighted intercept of the haptic intensity. $U_1$ and $U_2$ may denote independent variables related to the user information (e.g., an age, a gender, and the like) included in the user profile data and $W_{11}$ and $W_{12}$ may denote weights applied to the independent variables related to the user information. $P_1$ and $P_2$ may denote independent variables related to information (e.g., a type of the exercise program, the exercise intensity, and the like) related to the exercise program included in the exercise attribute data and $W_{21}$ and $W_{22}$ may weights applied to the independent variables related to the exercise program information. $S_1$ and $S_2$ may denote independent variables related to sensor information (e.g., motion information, heart rate information, and the like) included in the sensor data and $W_{31}$ and $W_{32}$ may denote weights applied to the independent variables related to the sensor information. In this case, the number of independent variables included in Equation 1 may be one or more or "0" respectively for the user information, the information related to the exercise program, and the sensor information. For example, only the independent variables for one of the user profile data, the exercise attribute data, and the sensor data may be used to determine the first boundary value $X_L$ of Equation 1.

The mathematical model for determining the second boundary value in the haptic intensity determination model may be the same as Equation 2 shown below.

[Equation 2]

$$X_U = X_L + K \times X_L$$

In this case, $X_L$ may denote the first boundary value (assuming that the first boundary value is the lower limit) of the threshold range of the haptic intensity and $X_U$ may denote the second boundary value (assuming that the second boundary value is the upper limit) of the threshold range of the haptic intensity. K may denote a weight applied to the first boundary value $X_L$ for determining the second boundary value $X_U$.

When at least one of the profile data of the user, the exercise attribute data, and the sensor data, the threshold range of the haptic intensity may be determined based on Equations 1 and 2. The threshold range of the determined haptic intensity may correspond to the haptic intensity range that is considered that the user is able to perceive the haptic feedback and may not feel discomfort by the haptic feedback.

In operation 840, the electronic device 210 may determine the haptic intensity of the haptic feedback based on the threshold range of the haptic intensity determined in operation 830. The electronic device 210 may determine the haptic intensity of the haptic feedback to be provided to the user within the threshold range of the determined haptic intensity. In an embodiment, the operation of determining the haptic intensity may include an operation of extracting a plurality of haptic intensity values in the threshold range of the haptic intensity and an operation of determining one of the extracted haptic intensity values to be the haptic intensity value of the haptic feedback to be provided to the user. For example, the plurality of haptic intensity values may be extracted from the threshold range by dividing the threshold range into n (n is a natural number greater than 2) parts. n may be the number that the user is able to perceive a change in the haptic intensity when the threshold range is divided into n parts. Among the extracted haptic intensity values, the smallest value, the median value (e.g., an intermediate value between the first boundary value and the second boundary value of the threshold range), or the largest value may be determined to be the haptic intensity value of the haptic feedback to be provided to the user. In an embodiment, when the exercise intensity increases, the electronic device 210 may gradually increase the haptic intensity within the threshold range and when the exercise intensity decreases, the electronic device 210 may gradually decrease the haptic intensity within the threshold range. The haptic intensity may discretely or continuously change.

In operation 850, the electronic device 210 may determine whether a haptic feedback provision event has occurred while the user performs the exercise program. For example, in at least one of a case in which a hip joint angle according to the exercise posture of the user reaches a reference angle and a case in which the exercise posture of the user deviates from a reference posture, it may be determined that the haptic feedback provision event has occurred. In addition, the haptic feedback provision event may occur in the case of adjusting the exercise speed (e.g., the walking speed), adjusting left-and-right gait balance, notification of switching between a resistance mode and an assistance mode of the wearable device 100, notification of exercise goal achievement, and notification of a collision with a surrounding object. When it is determined that the haptic feedback provision event does not occur (e.g., "No" in operation 850), the electronic device 210 may restart from operation 820.

When it is determined that the haptic feedback provision event has occurred (e.g., "Yes" in operation 850), in operation 860, the electronic device 210 may transmit a control signal to control to provide the user with the haptic feedback at the haptic intensity determined in operation 840 to the wearable device 100. A haptic pattern (e.g., a vibration pattern of a haptic actuator) determined for each haptic feedback provision event may exist and the electronic device 210 may transmit a control signal to control to generate a haptic pattern corresponding to the occurred haptic feedback provision event to the wearable device 100. The transmitted control signal may include information about the haptic intensity to be applied to each haptic actuator included in the wearable device 100. The wearable device 100 may set the haptic intensity of the haptic actuator based on the control signal received from the electronic device 210 and may output the haptic feedback in the haptic pattern corresponding to the haptic feedback provision event at the set haptic intensity. A maximum or high intensity of the haptic intensity shown in the haptic pattern may be set to the haptic intensity included in the control signal.

**In** operation 870, the electronic device 210 may determine whether the user has perceived the haptic feedback. Whether the user has perceived the haptic feedback may be determined based on whether the user performed an operation corresponding to the haptic feedback after the haptic feedback is provided. It may be determined that the user has perceived the haptic feedback when the user performs an operation according to the intention delivered by the haptic feedback. When the user does not perform an operation corresponding to the haptic feedback after the haptic feedback is provided, it may be determined that the user does not perceive the haptic feedback. For example, in a case where the user perceives that haptic feedback in a specific haptic pattern is an instruction to increase the walking speed when the haptic feedback is provided, it may be determined that the user does not perceive the haptic feedback when the haptic feedback is provided but the user does not increase the walking speed. When the user increases the walking speed after the haptic feedback is provided, it may be determined that the user has perceived the haptic feedback.

For example, whether the user has perceived the haptic feedback may be determined based on perception assessment information on haptic feedback input by the user. After the haptic feedback is provided, the electronic device may ask the user whether the user has perceived the haptic feedback through a guiding voice or an application. The user may input the perception assessment information regarding whether the user has perceived the haptic feedback. The perception assessment information may include, for example, perception of the haptic feedback and/or the intensity of the haptic feedback.

When it is determined that the user does not perceive the haptic feedback (e.g., "No" in operation 870), in operation 880, the electronic device 210 may adjust the threshold range of the haptic intensity. Parameters of the haptic intensity determination model may be updated during the adjustment of the threshold range. For example, the parameters of the haptic intensity determination model may include weights used to determine the first boundary value of Equation 1 and/or a weight applied to the first boundary value to determine the second boundary value of Equation 2. When it is determined that the user iteratively fails to perceive the haptic feedback, the electronic device 210 may adjust the haptic intensity of the haptic feedback until the user performs an operation when perceiving the haptic feedback. For example, the electronic device 210 may gradually increase the haptic intensity of the haptic feedback until the user performs an operation when the user perceives the haptic feedback. The electronic device 210 may determine the haptic intensity increased until the user perceives the haptic feedback and performs an operation intended by the haptic feedback to be the haptic intensity corresponding to the first boundary value by gradually increasing the haptic intensity of the haptic feedback.

Since the user may feel discomfort when the haptic intensity exceeds a predetermined level and the user may not be able to perceive the haptic feedback when the haptic intensity is too low, it may be important to determine an appropriate haptic intensity of the haptic feedback. On the other hand, a minimum and/or low intensity of the haptic intensity for perceiving the haptic feedback may vary depending on a user characteristic and/or an exercise condition. The electronic device 210 may adjust the haptic intensity of the haptic feedback personalized to the user based on the user characteristic and/or the exercise condition, and thereby the usability may be improved.

Although the embodiment describes that the electronic device 210 determines the threshold range of the haptic intensity of the haptic feedback and the haptic intensity within the threshold range, the wearable device 100 and/or a server (e.g., the server 230 of FIG. 2) other than the electronic device 210 may determine the threshold range of the haptic intensity of the haptic feedback and the haptic intensity within the threshold range according to the operations described above.

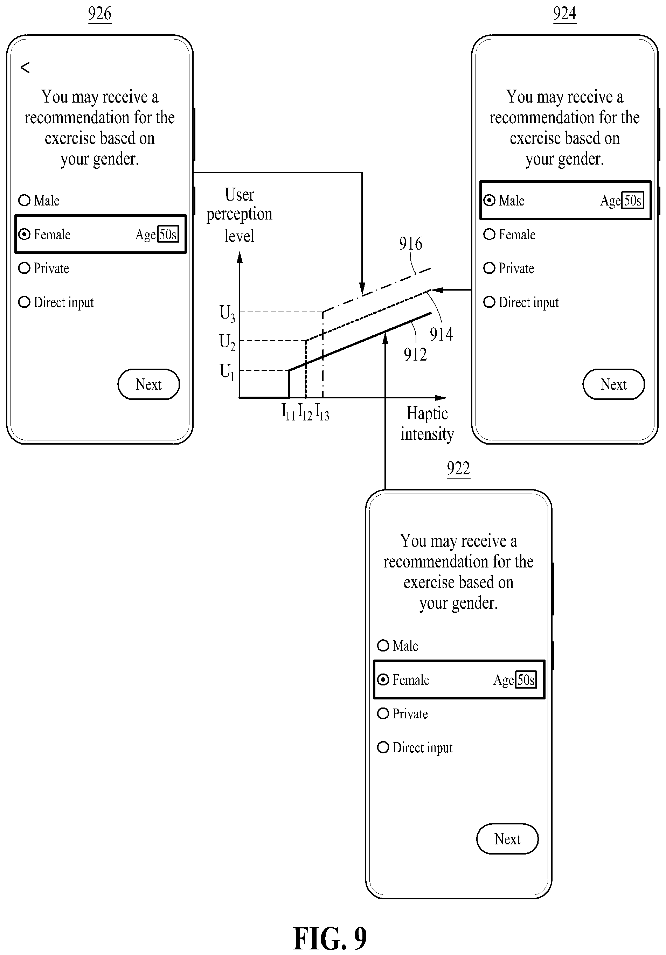

FIG. 9 is a diagram illustrating a difference in perception of haptic intensity based on a user characteristic according to an embodiment.

A minimum/low intensity (a haptic threshold value) of the haptic intensity at which haptic feedback is felt may vary between users depending on a user characteristic. Referring to FIG. 9, graphs 912, 914, and 916 showing a change in a perception level of a user with respect to the haptic intensity based on a user profile (e.g., a gender and an age) are illustrated. The graph 912 may show a change in user perception level based on the haptic intensity in a case 922 where the user profile is selected to female in 20s. In this case, the user may start to feel the haptic feedback at a haptic threshold value $I_{11}$ with the perception level $U_1$. The graph 914 may show a change in user perception level based on the haptic intensity in a case 924 where the user profile is selected to male in 50s. In this case, the user may start to feel the haptic feedback at a haptic threshold value $I_{12}$ with the perception level $U_2$. The graph 916 may show a change in user perception level based on the haptic intensity in a case 926 where the user profile is selected to female in 50s. In this case, the user may start to feel the haptic feedback at a haptic threshold value $I_{13}$ with the perception level $U_3$. The division of the user profile described as an example is merely an example and the division of the user profile may be further divided or differently divided. As shown in the haptic threshold values $I_{11}$, $I_{12}$, and $I_{13}$ shown in the graphs 912, 914, and 916, the haptic threshold value may typically increase as the age increases and a male group may tend to have a higher haptic threshold value than a female group. Accordingly, it may be preferable to adjust the haptic intensity of the haptic feedback by considering the user characteristics.

According to an embodiment, when determining the threshold range of the haptic intensity, the electronic device 210 and/or the wearable device 100 may determine the lower limit of the threshold range of the haptic intensity to be high as the age of the user increases and/or may determine the lower limit of the threshold range of the haptic intensity that is set when the user is male to be higher than the lower limit of the threshold range of the haptic intensity that is set when the user is female.

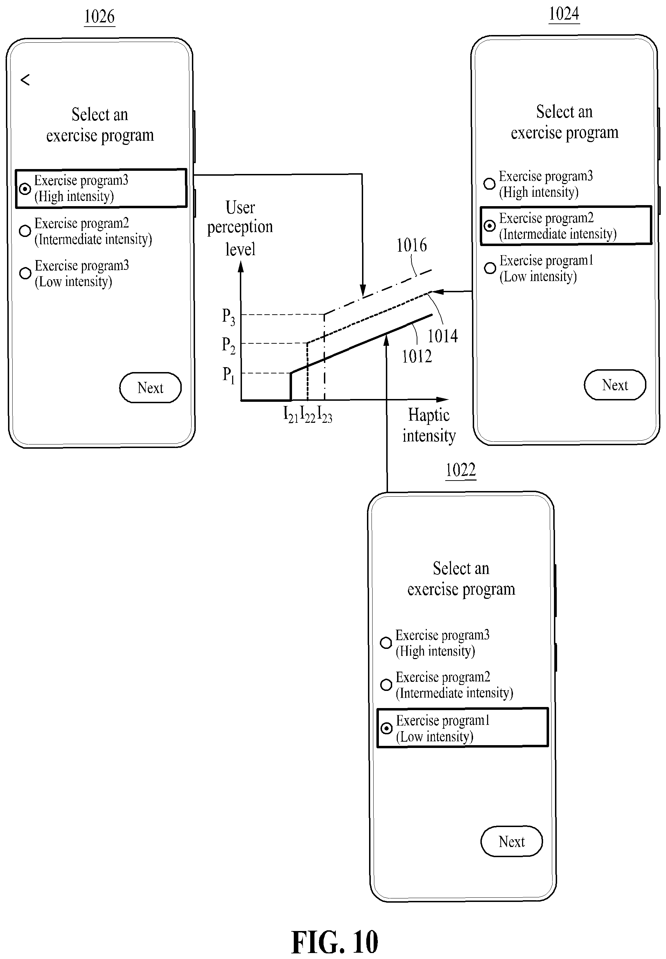

FIG. 10 is a diagram illustrating a difference in perception of haptic intensity based on the exercise intensity of an exercise program according to an embodiment.

The haptic intensity value, which is the minimum and/or low intensity of the haptic intensity at which the user feels the haptic feedback according to the exercise intensity of the exercise program, may vary between users. Referring to FIG. 10, graphs 1012, 1014, and 1016 showing a change in a perception level of a user with respect to the haptic intensity according to the exercise intensity of the exercise program are illustrated. The graph 1012 may show a change in the user perception level according to the haptic intensity in a case 1022 where a low-intensity exercise program is performed. In this case, the user may start to feel the haptic feedback at a haptic threshold value $I_{21}$ with the perception level $P_1$. The graph 1014 may show a change in the user perception level according to the haptic intensity in a case 1024 where an intermediateintensity exercise program is performed. In this case, the user may start to feel the haptic feedback at a haptic

threshold value $I_{22}$ with the perception level $P_2$. The graph 1016 may show a change in the user perception level according to the haptic intensity in a case 1026 where a highintensity exercise program is performed. In this case, the user may start to feel the haptic feedback at a haptic threshold value $I_{23}$ with the perception level $P_3$. The division of the exercise intensity described as an example is merely an example and the division of the exercise intensity may be further divided or differently divided. As shown in the haptic threshold values $I_{21}$, $I_{22}$, and $I_{23}$ shown in the graphs 1012, 1014, and 1016, the haptic threshold value may typically tend to increase as the exercise intensity increases. Accordingly, it may be preferable to adjust the haptic intensity of the haptic feedback by considering the exercise intensity of the exercise program.

According to an embodiment, when determining a threshold range of haptic intensity, the electronic device 210 and/or the wearable device 100 may determine the lower limit of the threshold range of the haptic intensity to be high as the exercise intensity increases by considering the tendency described above.

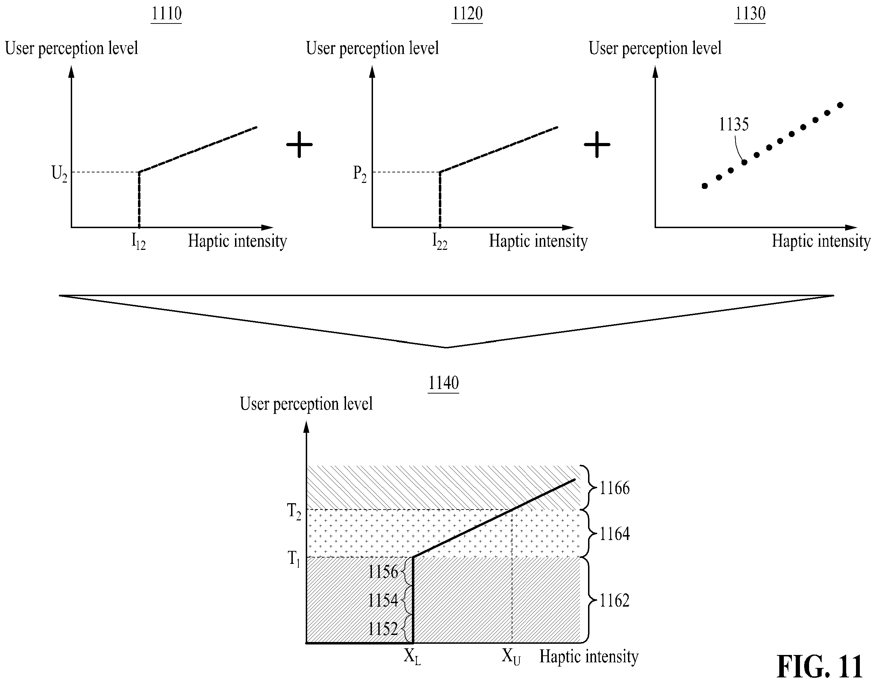

FIG. 11 is a diagram illustrating the determination of a threshold range for haptic intensity of haptic feedback according to an embodiment.

In an embodiment, a haptic threshold value of haptic intensity of haptic feedback that is finally perceptible by a user may be defined as a haptic threshold value derived based on at least one of a user characteristic, an exercise attribute related to an exercise program, and a sensor value. At haptic intensity greater than or equal to the haptic threshold value, it may be preferable to provide haptic feedback within an interval in which a user perceives haptic feedback without discomfort.

Referring to FIG. 11, graphs 1110, 1120, and 1130 showing a change in a user perception level with respect to haptic intensity respectively based on a user characteristic, exercise intensity of an exercise program, and sensor data (e.g., a sensor value of an inertial sensor and/or an angle sensor of the wearable device 100) are illustrated. A change in a user perception level with respect to haptic intensity that considers a user characteristic and an exercise condition of a specific user may be derived as shown in the graph 1140 by combining the effects of corresponding factors. The graph 1140 may assume that the graph 1140 reflects a perception level of the user according to the haptic intensity when the sensor has a sensor value 1135.

Referring to the graph 1140, an interval related to haptic feedback perception of the user may be divided into an interval 1162 in which the user is not able to perceive the haptic feedback, an interval 1164 in which the user perceives the haptic feedback without feeling discomfort, and an interval 1166 in which the user feels discomfort about the haptic feedback. The user may begin perceiving the haptic feedback at a haptic threshold value $X_L$ with a perception level $T_1$ and, when the haptic intensity exceeds a haptic intensity value $X_U$, the user may begin feeling discomfort. The haptic threshold value $X_L$ may be determined by a contribution 1152 based on a user characteristic, a contribution 1154 based on an exercise attribute of an exercise program, and a contribution 1156 based on sensor data. The contributions may respectively represent, for example, degrees of effects of the user characteristic, the exercise attribute of the exercise program, and the sensor data on the determination of a first boundary value (corresponds to the haptic threshold value $X_L$) in Equation 1. The contributions 1154 and 1156 may represent contributions depending on exercise conditions.

According to an embodiment, the electronic device 210 and/or the wearable device 100 may determine a threshold range of the haptic intensity of the haptic feedback based on at least one of user profile data, exercise attribute data of the exercise program, and the sensor data. The threshold range of the determined haptic intensity may be, for example, determined through Equations 1 and 2, and may correspond to the interval 1164 in which the user perceives the haptic feedback but does not feel discomfort in the graph 1140. The electronic device 210 and/or the wearable device 100 may determine a threshold range of the haptic intensity at which the user is expected to perceive the haptic feedback while the user does not feel discomfort due to the haptic feedback by considering the user characteristic and/or the exercise condition.

The embodiment describes based on an example of determining the threshold range of the haptic intensity based on all three of the user profile data, the exercise attribute data of the exercise program, and the sensor data. However, the embodiment is not limited thereto. For example, the threshold range of the haptic intensity may be determined based on one of the user profile data, the exercise attribute data of the exercise program, and the sensor data, or any combination thereof.

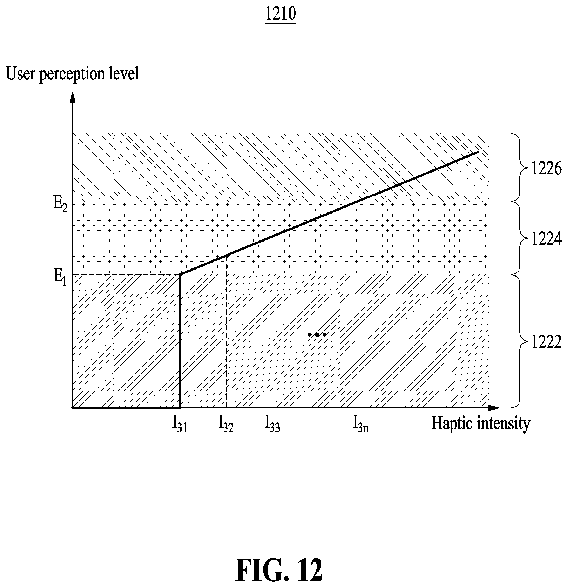

FIG. 12 is a diagram illustrating the determination of haptic intensity of haptic feedback in a threshold range for the haptic intensity according to an embodiment.

Referring to FIG. 12, a graph 1210 may show a change in a user perception level with respect to haptic intensity of the user. An interval related to the haptic feedback perception of the user may be divided into an interval 1222 in which the user is not able to perceive the haptic feedback, an interval 1224 in which the user perceives the haptic feedback without feeling discomfort, and an interval 1226 in which the user feels discomfort about the haptic feedback. It may be assumed that the user may begin perceiving haptic feedback at a haptic threshold value $I_{31}$ with a perception level $E_1$, the user may feel a perception level $E_2$ when a haptic intensity reaches a haptic intensity value $I_{3n}$, and the user may feel discomfort when the haptic intensity exceeds the perception level $E_2$.

The electronic device 210 and/or the wearable device 100 may determine the interval 1224 by considering the user characteristic and/or the exercise condition. The interval 1224 may correspond to a threshold range of the haptic intensity. n haptic intensity values $I_{31}$, $I_{32}$, $I_{33}$, ... , and $I_{3n}$ may be extracted from the threshold range to be perceived by the user as a haptic pattern is classified based on a scenario to deliver the haptic feedback to the user. Haptic feedback at one of the extracted haptic intensity values $I_{31}$, $I_{32}$, $I_{33}$, ... , and $I_{3n}$ may be provided to the user based on the scenario. For example, the smallest value $I_{31}$, the intermediate value, or the largest number $I_{3n}$ of the extracted haptic intensity values $I_{31}$, $I_{32}$, $I_{33}$, ... , and $I_{3n}$ may be determined to be the haptic intensity value of the haptic feedback to be provided to the user. In an embodiment, when the exercise intensity increases during the exercise of the user, the electronic device 210 and/or the wearable device 100 may gradually increase the haptic intensity within the interval 1224 and may gradually decrease the haptic intensity within the interval 1224 when the exercise intensity decreases.

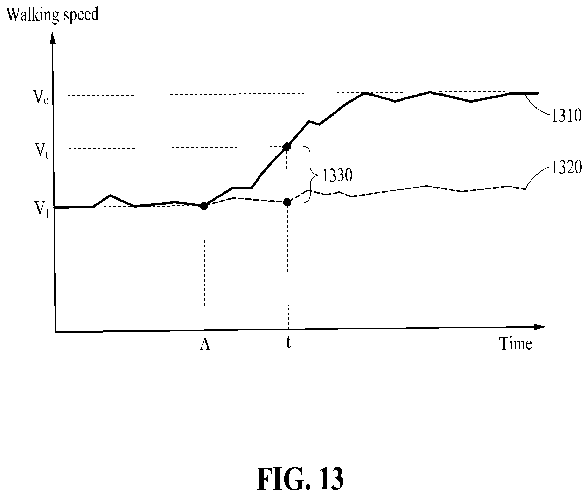

FIG. 13 is a diagram illustrating a method of determining whether to perceive haptic feedback of a user according to an embodiment.

The electronic device 210 and/or the wearable device 100 may determine whether the user perceives the haptic feedback after the haptic feedback is output. A process of determining whether the user perceives the haptic feedback may be required to determine whether the haptic threshold value at the currently set haptic intensity is appropriate (e.g., whether the haptic threshold value is low to be perceived by the user).

For example, whether the user has perceived the haptic feedback may be estimated based on whether the user performed an operation corresponding to the haptic feedback after the haptic feedback is output. It may be determined that the user has perceived the haptic feedback when the user performs an operation according to the intention delivered by the haptic feedback. When the user does not perform an operation corresponding to the haptic feedback after the haptic feedback is provided, it may be determined that the user does not perceive the haptic feedback.

Referring to FIG. 13, it may be assumed that when haptic feedback is provided while a user performs walking exercise at an average walking speed $V_1$, the user knows the walking speed needs to increase to a target walking speed $V_0$. An operation guide according to the haptic feedback may be provided to the user before starting the exercise or during the exercise. When haptic feedback is provided by the wearable device 100 at time A and the walking speed of the user is measured as a graph 1310, it may be determined that the user perceives the haptic feedback. Alternatively, when the walking speed of the user does not significantly change as a graph 1320 after the time A at which the haptic feedback is provided, it may be determined that the user does not perceive the haptic feedback. For example, whether the haptic feedback is perceived may be determined by comparing indicators (e.g., the walking speed, a hip joint angle, and the like) related to the exercise at time t in the case where the haptic feedback is output and in the case where the haptic feedback is not output. When the indicator does not significantly change even when the haptic feedback is output, it may be determined that the user does not perceive the haptic feedback. When it is determined that the user does not perceive the haptic feedback, a process of checking whether the user perceives the haptic feedback may be performed.

In an embodiment, the perception of the haptic feedback may be determined through a time series analysis method using an autoregressive integrated moving average (ARIMA) model. In the ARIMA model, an equation for representing an ARIMA-Intervention model for determining an effect of a specific event X when the specific event X has occurred, such as an output of the haptic feedback.

[Equation 3]

$$V_t - V_1 = \phi_1(V_{t-1} - V_1) + \phi_2(V_{t-2} - V_1) + \ldots + \phi_p(V_{t-p} - V_1) + \beta X_t + \varepsilon_t - \theta_1\varepsilon_{t-1} - \theta_2\varepsilon_{t-2} - \ldots - \theta_q\varepsilon_{t-q}$$

In this case, $V_t$ may denote walking speed corresponding to a time-series data value at the time t. $V_1$ may denote average walking speed before the haptic feedback corresponding to an average of an entirety of time-series data. $\phi_1$, $\phi_2$, ... , $\phi_p$ may represent auto-regression (AR) parameters of the ARIMA-Intervention model. $\theta_1$, $\theta_2$, ... , $\theta_q$ may represent moving average (MA) parameters of the ARIMA-Intervention model. $X_t$ may denote a binary variable representing an event X. For example, $X_t$ may have a value of "1" when the event X occurs and may have a value of "0" at other times. $\varepsilon t$ may represent a white noise element and $\beta$ may represent a regression coefficient. When an event of outputting haptic feedback through the ARIMA-Intervention model described above has occurred, an effect of the event represented by $V_t - V_1$ 1330 may be numerically determined. For example, when $V_t - V_1$ 1330 is greater than or equal to a predetermined threshold value, it may be determined that the user perceives the haptic feedback and when $V_t - V_1$ 1330 is less than the predetermined threshold value, it may be determined that the user does not perceive the haptic feedback.

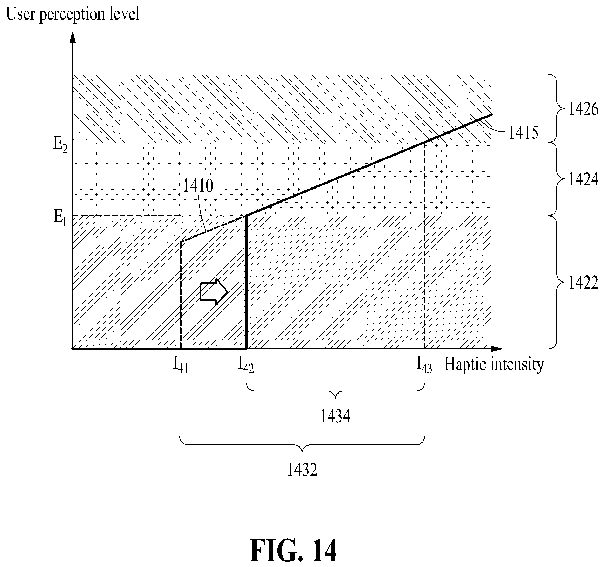

FIG. 14 is a diagram illustrating the adjustment of a threshold range for haptic intensity according to an embodiment.

When haptic feedback is output through the wearable device 100 but it is determined that the user does not perceive the haptic feedback, a process of adjusting a threshold range of haptic intensity may be performed. The process of adjusting the threshold range may be, for example, automatically performed or may be performed when the user explicitly indicates that the user fails to perceive the haptic feedback through a process of asking the user whether the user has perceived the haptic feedback.

In an embodiment, the process of adjusting the threshold range may gradually increase the haptic threshold value of the haptic intensity until the user is able to perceive the haptic feedback or may be performed by explicitly asking the user whether the user has satisfied the adjusted haptic intensity after adjusting the haptic intensity.

Referring to FIG. 14, it may be assumed that an interval related to haptic feedback perception of a predetermined user is divided into an interval 1422 in which the user is not able to perceive the haptic feedback, an interval 1424 in which the user perceives the haptic feedback without feeling discomfort, and an interval 1426 in which the user feels discomfort about the haptic feedback. It may be assumed that the user may begin perceiving haptic feedback at a haptic threshold value $I_{42}$ with a perception level $E_1$, the user may feel a perception level $E_2$ when a haptic intensity reaches a haptic intensity value $I_{43}$, and the user may feel discomfort when the haptic intensity exceeds the perception level $E_2$.

It may be assumed that a threshold range of haptic intensity at the beginning is determined to be a threshold range 1432, as shown in graph 1410, and haptic feedback at haptic intensity $I_{41}$ corresponding to a haptic threshold value of the threshold range 1432 is provided to the user. Because the haptic intensity $I_{41}$ does not provide perception intensity included in the intervals 1424 and 1426 in which the user is able to perceive the haptic feedback, the user may not be able to perceive the haptic feedback at the haptic intensity $I_{41}$. When it is determined that the user fails to perceive the haptic feedback, a process of adjusting the threshold range of the haptic intensity may be performed. In an embodiment, the electronic device 210 and/or the wearable device 100 may gradually increase the haptic threshold value of the haptic intensity from the haptic intensity $I_{41}$ until the user confirms that the user has perceived the haptic feedback. When the perception of the haptic feedback by the user is confirmed, the electronic device 210 and/or the wearable device 100 may determine the haptic intensity at the time of confirming that the user has perceived the haptic feedback to be the haptic threshold value and may set the threshold range of the haptic intensity again based on the determined haptic intensity value. For example, when haptic feedback at the haptic intensity $I_{42}$ is provided and the user confirms that the user has perceived the haptic feedback, the threshold range of the haptic intensity may be adjusted to a threshold range 1434 based on the haptic intensity $I_{42}$. The graph 1415 may represent a graph of the change in the user's perceived haptic intensity as a result of the adjustment of the threshold interval 1434. The threshold range 1434 may correspond to an interval between the haptic intensity $I_{42}$ at which the user begins perceiving the haptic feedback and the haptic intensity $I_{43}$ that is the maximum or a high haptic intensity at which the user does not feel discomfort due to the haptic feedback.

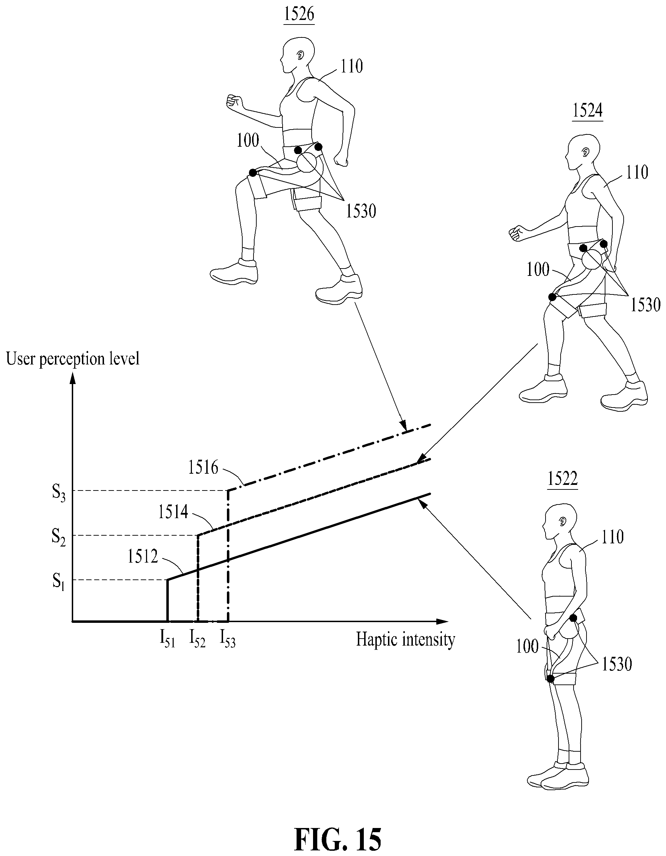

FIG. 15 is a diagram illustrating an example of adjusting haptic intensity based on sensor data according to an embodiment.

While the exercise is performed by the user 110, a haptic threshold value, which is the minimum intensity of haptic intensity at which the user 110 feels haptic feedback, may vary depending on an exercise state of the user 110. When a motion value (e.g., acceleration, velocity, and hip joint angular velocity) per hour or a heart rate of the user 110 is high, it may be estimated that the user 110 is in an exercise state in which the user 110 actively performs the exercise and when the motion value per hour or the heart rate of the user 110 is low, it may be estimated that the user is in an exercise state in which the user 110 does not actively perform the exercise.

Referring to FIG. 15, graphs 1512, 1514, and 1516 showing changes in a perception level of the user 110 with respect to haptic intensity according to the exercise intensity are illustrated. The graph 1512 may show a change in a perception level of the user 110 according to the haptic intensity in an exercise state 1522 in which the user 110 stops the exercise. In this case, the user 110 may start feeling the haptic feedback at a perception level $S_1$ at a haptic intensity value $I_{51}$. The graph 1514 may show a change in the perception level of the user 110 according to the haptic intensity when the user 110 is in a low-intensity exercise state (performing the exercise but the exercise is not active) 1524. In this case, the user 110 may start feeling the haptic feedback at a perception level $S_2$ at a haptic intensity value $I_{52}$. The graph 1516 may show a change in the perception level of the user 110 according to the haptic intensity when the user 110 is in a highintensity exercise state (actively performing the exercise) 1526. In this case, the user 110 may start feeling the haptic feedback at a perception level $S_3$ at a haptic intensity value $I_{53}$. The division of the exercise state described as an example is merely an example and the division of the exercise state may be further divided or differently divided. As shown in the haptic threshold values $I_{51}$, $I_{52}$, and $I_{53}$ shown in the graphs 1512, 1514, and 1516, the haptic threshold value may typically tend to increase as the exercise is more actively performed. Accordingly, it may be preferable to adjust the haptic intensity of the haptic feedback by considering the exercise state of the user 110.

According to an embodiment, by considering the tendency described above, the electronic device 210 and/or the wearable device 100 may adjust the haptic intensity of the haptic feedback provided by a haptic actuator 1530 of the wearable device 100 based on a real-time exercise state (e.g., a change in the exercise intensity) of the user 110 measured by a sensor. When the exercise intensity increases during the exercise, the electronic device 210 and/or the wearable device 100 may gradually increase the haptic intensity within a threshold range with respect to the haptic intensity and when the exercise intensity decreases, the electronic device 210 and/or the wearable device 100 may gradually decrease the haptic intensity within the threshold range with respect to the haptic intensity. The haptic intensity may discretely or continuously change depending on the exercise state.

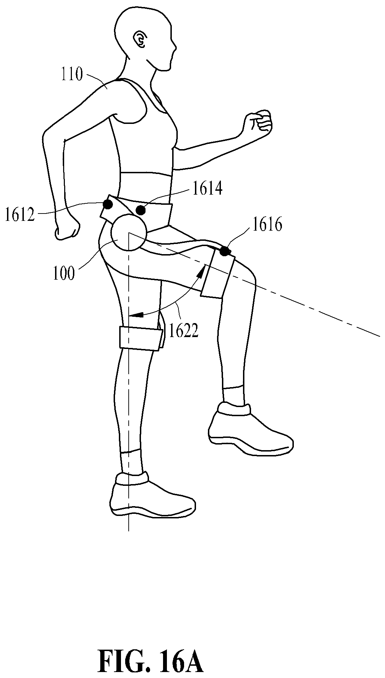

FIGS. 16A to 16E are diagrams illustrating exercise situations in which a haptic feedback provision event occurs according to an embodiment.

Referring to FIG. 16A, when the user 110 performs exercise using both legs, such as walking, the haptic feedback may be provided to guide walking speed, a tempo of motions of both legs, and/or a side-to-side balance. For example, when a haptic pattern of haptic feedback corresponding to target walking speed is provided to the user 110 through at least one haptic actuator 1612, 1614, or 1616 included in the wearable device 100, the user 110 may intuitively perceive the walking tempo according to the target walking speed through the haptic pattern. The haptic feedback may be, for example, provided by one haptic actuator or haptic actuators arranged in thigh fasteners of both legs (e.g., the first thigh fastener 2 and the second thigh fastener 1 of FIG. 3). In an embodiment, when using one haptic actuator, the tempo of walking may be guided by haptic feedback and when using haptic actuators arranged in thigh fasteners of both legs, respective walking tempos of left and right legs may be separately guided.

In an embodiment, when an exercise program of walking exercise is performed and the target exercise speed of the walking exercise increases, a cycle of the haptic pattern of the haptic feedback provided through the at least one haptic actuator 1612, 1614, or 1616 of the wearable device 100 may decrease. The user 110 may intuitively perceive that the walking speed needs to increase according to the haptic pattern by decreasing the cycle of the haptic pattern.

In an embodiment, the haptic feedback may be provided to guide an exercise posture of the user 110. By providing the haptic feedback when the exercise posture of the user 110 reaches the target posture, the user may be intuitively aware of reaching the target posture. For example, it may be assumed that the user 110 performs a knee-up exercise that strengthens the lower body strength by raising a thigh up. When an angle formed by a right thigh and a left thigh reaches a target angle 1622 while the user performs an exercise posture of raising up a right thigh, the haptic feedback may be provided to the user 110 through a haptic actuator 1616 of the right thigh. The angle formed by both thighs may be determined based on sensor data obtained by an angle sensor (e.g., the first angle sensor 524 and the second angle sensor 524-1 of FIG. 5B) of the wearable device 100. When an angle formed by the right thigh and the left thigh reaches the target angle 1622 while the user performs an exercise posture of raising up the left thigh, the haptic feedback may be provided to the user 110 through a haptic actuator (not shown) of the left thigh.

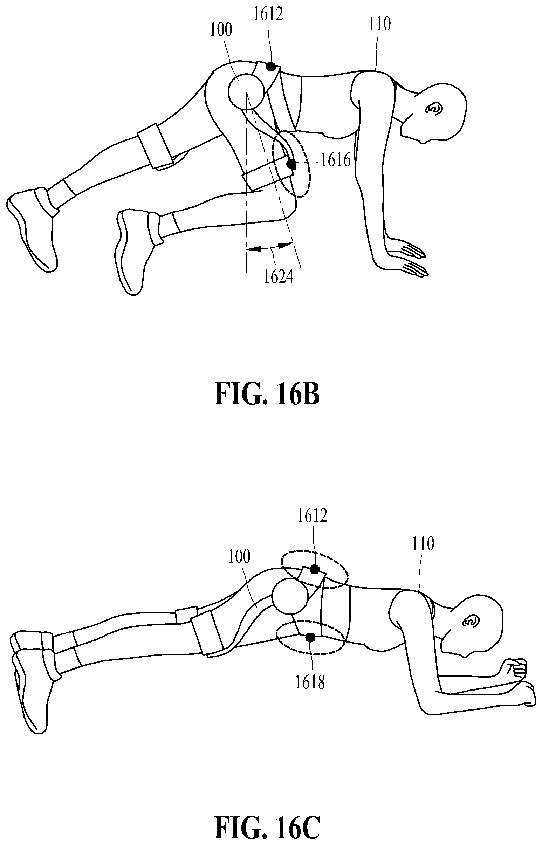

FIG. 16B illustrates an exercise posture when the user 110 performs a mountain climber exercise according to an embodiment. A bending angle of a hip joint of the user 110 may be measured by an angle sensor included in the wearable device 100 and when the measured bending angle reaches a target angle 1624, haptic feedback may be provided through a haptic actuator (e.g., the haptic actuator 1616). The user 110 may be able to intuitively perceive that the bending angle of the hip joint reaches the target angle 1624 through the haptic feedback and a preferable exercise posture may be guided to the user 110.

FIG. 16C illustrates an exercise posture when the user 110 performs a plank exercise according to an embodiment. A torso posture of the user 110 may be measured by an inertial sensor (e.g., the inertial sensor 522 of FIG. 5B) included in the wearable device 100. In an embodiment, when a torso position of the user 110 moves upward beyond an acceptable range, haptic feedback may be output to a lumbar area of the user 110 through the haptic actuator 1612 and when the torso position of the user 110 moves downward beyond the acceptable range, the haptic feedback may be output to an abdominal area of the user 110 through the haptic actuator 1618. The user 110 may intuitively perceive that the exercise posture of the plank exercise is incorrect through the haptic feedback. As described above, the haptic feedback may help the user 110 to maintain a consistent exercise posture that the user 110 aims for.

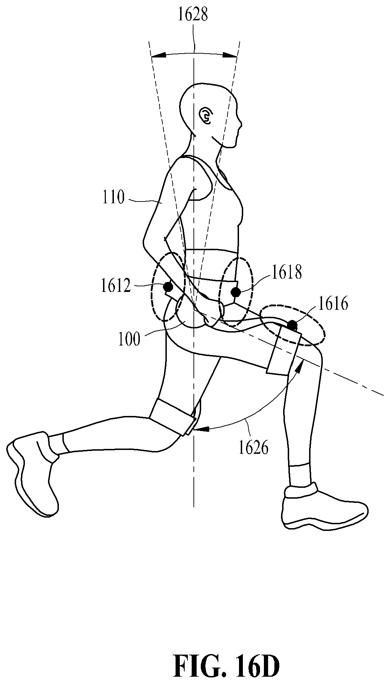

FIG. 16D illustrates an exercise posture when the user 110 performs lunge exercise according to an embodiment. A bending angle of a hip joint of the user 110 may be measured by an angle sensor included in the wearable device 100 and when the measured bending angle reaches a target angle 1626, haptic feedback may be provided through a haptic actuator (e.g., the haptic actuator 1616). The user 110 may intuitively perceive that the bending angle of the hip joint reaches the target angle 1626 through the haptic feedback. In addition, a torso position of the user 110 may be measured by an inertial sensor included in the wearable device 100. When the measured torso position deviates from an acceptable angle range 1628, the haptic feedback may be provided through a haptic actuator (e.g., the haptic actuators 1612 and 1618). For example, when the torso of the user 110 tilts forward and deviates the acceptable angle range 1628, the haptic feedback may be provided through the haptic actuator 1618 and the user 110 may intuitively perceive that the torso position deviates the acceptable angle range 1628 and tilts forward through the haptic feedback delivered to the abdominal area. When the torso of the user 110 tilts backward and deviates the acceptable angle range 1628, the haptic feedback may be provided through the haptic actuator 1612 and the user 110 may intuitively perceive that the torso position deviates the acceptable angle range 1628 and tilts backward through the haptic feedback delivered to the lumbar area. The haptic actuators 1612, 1616, and 1618 included in the wearable device 100 may simultaneously or individually provide the haptic feedback based on an exercise scenario. In addition, the haptic actuators 1612, 1616, and 1618 may provide haptic feedback having the same intensity or different intensities.

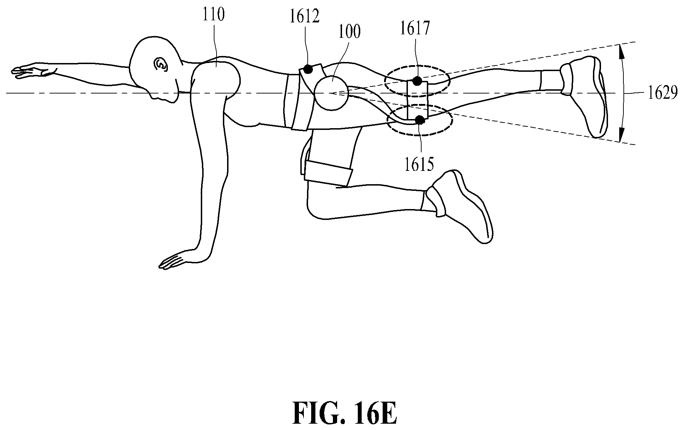

FIG. 16E illustrates an exercise posture when the user 110 performs bird dog exercise according to an embodiment. A leg posture of the user 110 may be measured by an angle sensor included in the wearable device 100. In an embodiment, when the leg position of the user 110 moves upward beyond an acceptable range 1629, haptic feedback may be output to the back of the thigh of the user 110 through the haptic actuator 1617, and when the leg position of the user 110 moves downward beyond the acceptable range 1629, the haptic feedback may be output to the front of the thigh of the user 110 through the haptic actuator 1615. In the bird dog exercise, it may be important for the user 110 to maintain the legs horizontally, and the user 110 may intuitively perceive whether the user 110 maintains their legs horizontally through the haptic feedback.

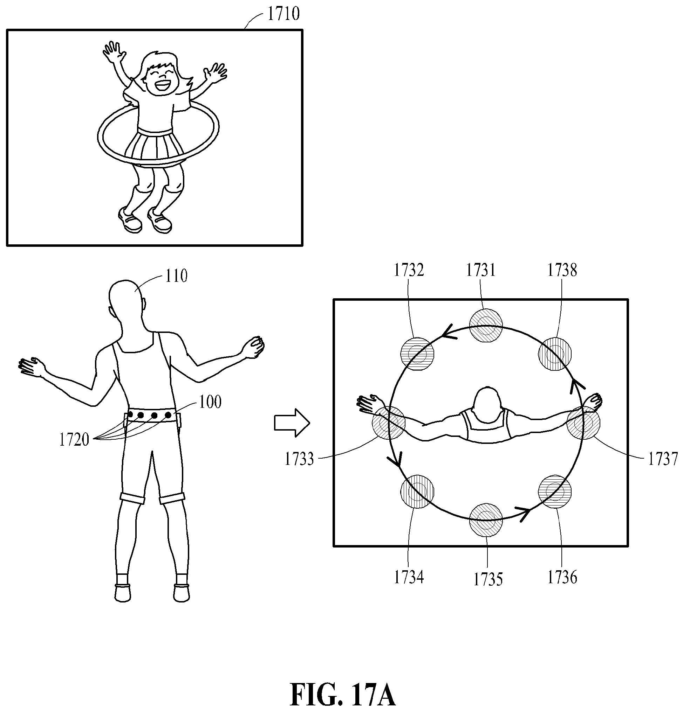

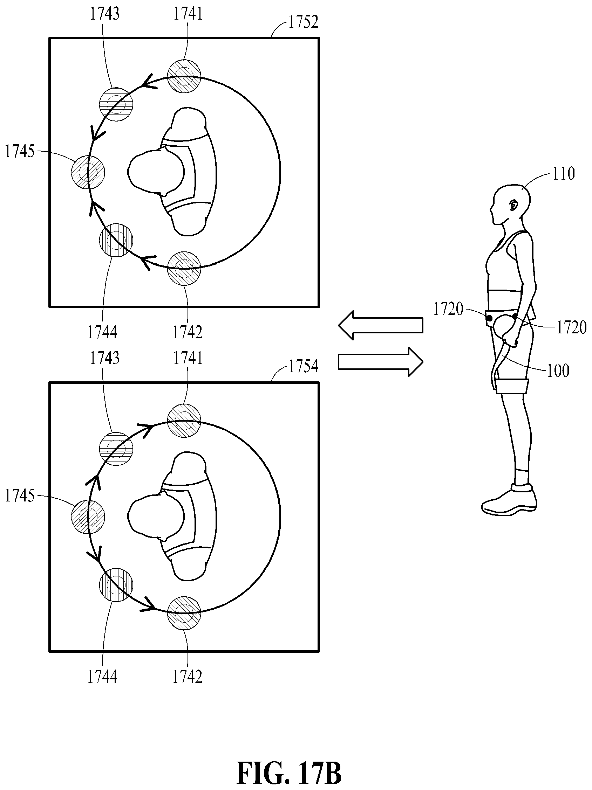

FIGS. 17A and 17B are diagrams illustrating applications using haptic feedback according to an embodiment.

The haptic feedback may be used by various applications. Referring to FIG. 17A, the user 110 may wear the wearable device 100 and may perform an exercise program, such as a virtual hula hoop exercise, based on hula hoop exercise content displayed on a display device 1710 (e.g., a television (TV) or a monitor). In an embodiment, when haptic actuators 1720 are provided in a base body and a waist support frame of the wearable device 100, a waist direction of the user 110 may be estimated based on sensor data obtained by an inertial sensor and the sense of reproduction of performing a hula hoop exercise may be provided to the user 110 by controlling the haptic feedback output by each of the haptic actuators 1720 based on the estimated waist direction. The haptic actuators 1720 may be spaced apart from each other on the abdominal circumference of the user 110. When the user 110 wears the wearable device 100 and performs a waist rotation exercise, such as circling the hula hoop, the haptic actuators may be controlled to sequentially output the haptic feedback from the haptic actuator corresponding to the outermost direction. For example, when the user 110 performs a waist rotation exercise and the waist direction of the user approaches a position 1731, the haptic feedback may be output through a haptic actuator disposed at the position 1731, and when the waist direction of the user 110 approaches a position 1732, the haptic feedback may be output through the haptic actuator disposed at the position 1732. Thereafter, when the waist direction of the user 110 sequentially approaches positions 1733, 1734, 1735, 1736, 1737, and 1738 based on the rotation order, the haptic feedback may be output through haptic actuators disposed at the positions 1733, 1734, 1735, 1736, 1737, and 1738, respectively. Thereafter, the haptic feedback may be sequentially output from the position 1731. Through the control, the user 110 may feel the sense of a circling hula hoop even if the user 110 does not actually spin a hula hoop.

Referring to FIG. 17B, the user 110 may wear the wearable device 100 and may perform abdominal breathing according to inspiration guidance based on haptic feedback. In an embodiment, when the haptic actuators 1720 of the wearable device 100 are disposed on the waist circumference of the user 110, a deep inhalation or exhalation may be guided through the sequential control of haptic feedback to the user 110. In a case 1752 of inducing inhalation, the haptic feedback may be firstly output through haptic actuators disposed at positions 1741 and 1742, and then the haptic feedback may be output through haptic actuators disposed at positions 1743 and 1744, and lastly, the haptic feedback may be output through a haptic actuator disposed at a position 1745 at the most forward position. Through the sequential output of haptic feedback, the tempo of deep inspiration and inhalation in a direction of expanding the abdomen may be guided to the user 110. In a case 1754 of inducing exhalation, the haptic feedback may be firstly output through the haptic actuator disposed at the position 1745 at the most forward position, and then the haptic feedback may be output through the haptic actuators disposed at the positions 1743 and 1744, and lastly, the haptic feedback may be output through the haptic actuators disposed at the positions 1741 and 1742. Through the sequential output of haptic feedback, the tempo of deep inspiration and exhalation in a direction of contracting the abdomen may be guided to the user 110.

It should be appreciated that various embodiments of the present disclosure and the terms used therein are not intended to limit the technological features set forth herein to particular embodiments and include various changes, equivalents, or replacements for a corresponding embodiment. In connection with the description of the drawings, like reference numerals may be used for similar or related components. It is to be understood that a singular form of a noun corresponding to an item may include one or more of the things, unless the relevant context clearly indicates otherwise. As used herein, "A or B," "at least one of A and B," "at least one of A or B," "A, B or C," "at least one of A, B and C," and "at least one of A, B, or C," may include any one of the items listed together in the corresponding one of the phrases, or all possible combinations thereof. Terms such as "1st" and "2nd," or "first" and "second" may be used to simply distinguish a corresponding component from other components, and do not limit the components in other aspects (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., by wire), wirelessly, or via at least a third element.

As used in connection with various embodiments of the disclosure, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC). Accordingly, each "module" in the present disclosure may include a circuit/circuitry.

The software may include a computer program, a piece of code, an instruction, or some combination thereof, to independently or uniformly instruct or configure the processing device to operate as desired. Software and data may be embodied permanently or temporarily in any type of machine, component, physical or virtual equipment, or computer storage medium or device capable of providing instructions or data to or being interpreted by the processing device. The software also may be distributed over network-coupled computer systems so that the software is stored and executed in a distributed fashion. The software and data may be stored by one or more non-transitory computer-readable recording mediums. Embodiments as set forth herein may be implemented as software including one or more instructions that are stored in a storage medium that is readable by a machine. For example, a processor of the machine may invoke at least one of the one or more instructions stored in the storage medium and execute it. This allows the machine to be operated to perform at least one function according to the at least one instruction invoked. The one or more instructions may include code generated by a compiler or code executable by an interpreter. The machine-readable storage medium may be provided in the form of a non-transitory storage medium. Here, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to embodiments may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to embodiments, each component (e.g., a module or a program) of the above-described components may include a single entity or multiple entities, and some of the multiple entities may be separately disposed in different components. According to embodiments, one or more of the above-described components may be omitted, or one or more other components may be added. Alternatively or additionally, a plurality of components (e.g., modules or programs) may be integrated into a single component. In such a case, according to embodiments, the integrated component may still perform one or more functions of each of the plurality of components in the same or similar manner as they are performed by a corresponding one of the plurality of components before the integration. According to embodiments, operations performed by the module, the program, or another component may be carried out sequentially, in parallel, repeatedly, or heuristically, or one or more of the operations may be executed in a different order or omitted, or one or more other operations may be added.

Although the present disclosure exemplifies and describes with reference to various embodiments, it shall be construed that various embodiments are for the illustrative purpose rather than limiting. It shall be further understood by those skilled in the art that various changes in forms and details may be made without departing from the true spirit and full scope of this disclosure including the scope of the attached claims and their equivalents. In addition, it shall be construed that the embodiments described herein may be used with other embodiments of the present disclosure.

**Claims**

1.   A haptic feedback control method for controlling haptic feedback of a wearable device(100), the method comprising:

when an exercise program is performed using the wearable device(100), receiving sensor data comprising motion information of a user from the wearable device(100);
determining a threshold range with respect to haptic intensity of the haptic feedback based on at least one of user profile data of the user, exercise attribute data of the exercise program, and the sensor data;
determining haptic intensity of haptic feedback to be provided to the user within the threshold range with respect to the determined haptic intensity; and
when a haptic feedback provision event occurs, transmitting a control signal configured to control to provide the user with haptic feedback at the determined haptic intensity.

2.   The method of claim 1, wherein the determining of the threshold range with respect to the haptic intensity comprises:

determining a first threshold value of the threshold range based on at least one of the user profile data of the user, the exercise attribute data of the exercise program, and the sensor data; and
determining a second threshold value of the threshold range at least by applying a weight to the determined first threshold value,
wherein the threshold range with respect to the haptic intensity corresponds to a range between the first threshold value and the second threshold value.

3.   The method of claim 1 or 2, wherein the determining of the threshold range with respect to the haptic intensity comprises:

as exercise intensity of the exercise program increases, determining a lower limit value of the threshold range with respect to the haptic intensity to be high; and/or
as an age of the user increases, determining a lower limit value of the threshold range with respect to the haptic intensity to be high.

4. The method of claim any one of claims 1-3, wherein a lower limit value of the threshold range with respect to the haptic intensity when the user is male is greater than a lower limit value of the threshold range with respect to the haptic intensity when the user is female.

5. The method of claim any one of claims 1-4, wherein the determining of the threshold range with respect to the haptic intensity comprises, when at least one of the exercise attribute data and the sensor data changes while performing the exercise program, adjusting the threshold range with respect to the determined haptic intensity.

6. The method of claim any one of claims 1-5, wherein the determining of the haptic intensity comprises:

extracting a plurality of haptic intensity values within the threshold range with respect to the haptic intensity; and determining one of the extracted haptic intensity values to be a haptic intensity value of haptic feedback to be provided to the user.

7. The method of claim any one of claims 1-6, further comprising:

determining whether the user perceives the haptic feedback; and
in response to determination that the user fails to perceive the haptic feedback, adjusting the threshold range with respect to the haptic intensity.

8. The method of claim any one of claims 1-7, wherein, at least one of a case in which a hip joint angle according to an exercise posture of the user reaches a reference angle and a case in which an exercise posture of the user deviates from a reference posture, the haptic feedback provision event occurs.

9. A non-transitory computer-readable storage medium storing instructions that, when executed by at least one processor, cause the at least one processor to individually and/or collectively perform the method of claim any one of claims 1-8.

10. An electronic device (210) comprising:

at least one processor(710) comprising processing circuitry; and
a communication module(730), comprising communication circuitry, configured to communicate with a wearable device(100) under control of the at least one processor(710),
wherein the at least one processor(710) is individually and/or collectively configured to:

when an exercise program is performed via the wearable device(100), receive sensor data, comprising motion information of a user, from the wearable device(100) through at least the communication module, determine a threshold range with respect to haptic intensity of haptic feedback based on at least one of user profile data of the user, exercise attribute data of the exercise program, and the sensor data, determine haptic intensity of haptic feedback to be provided to the user within the threshold range with respect to the determined haptic intensity, and
control to transmit a control signal configured to control to provide the user with haptic feedback at the determined haptic intensity through at least the communication module(730), based on occurrence of a haptic feedback provision event.

11. The electronic device (210) of claim 10, wherein at least one processor(710), comprising processing circuitry, is individually and/or collectively configured to:

determine a first threshold value of the threshold range based on at least one of the user profile data of the user, the exercise attribute data of the exercise program, and the sensor data,
determine a second threshold value of the threshold range at least by applying a weight to the determined first threshold value, and
wherein the threshold range with respect to the haptic intensity corresponds to a range between the first threshold value and the second threshold value.

12. The electronic device (210) of claim 10 or 11, wherein at least one processor(710), comprising processing circuitry, is individually and/or collectively configured to, when at least one of the exercise attribute data and the sensor data changes while performing the exercise program, adjust the threshold range with respect to the determined haptic intensity.

13. The electronic device (210) of claim any one of claims 10-12, wherein at least one processor(710), comprising processing circuitry, is individually and/or collectively configured to:

determine whether the user perceives the haptic feedback, and
in response to a determination that the user fails to perceive the haptic feedback, adjust the threshold range with respect to the haptic intensity.

14. The electronic device (210) of claim any one of claims 10-13, wherein at least one processor(710), comprising processing circuitry, is individually and/or collectively configured to, when an exercise program of walking exercise is performed and target exercise speed of the walking exercise increases, generate a control signal configured to decrease a cycle of a haptic pattern of haptic feedback provided through the wearable device(100).

15. A wearable device(100), comprising:

a driving module(530; 530-1), comprising a motor(534; 534-1), configured to generate torque;
a torque transmission frame(50; 55) configured to transmit the generated torque to a leg of a user;
a thigh fastener(1; 2) connected to the torque transmission frame(50; 55) and configured to connect the torque transmission frame(50; 55) to the leg of the user;
a sensor module(520), comprising a sensor, configured to obtain sensor data comprising motion information of the user when an exercise program using the wearable device(100) is performed;
a haptic module(560), comprising a haptic actuator, configured to provide haptic feedback to the user;
a communication module(516), comprising communication circuitry, configured to receive haptic intensity data comprising information about haptic intensity of the haptic feedback from an electronic device (210); and
a processor(512), comprising processing circuitry, configured to control haptic intensity generated by the haptic actuator based on information about the haptic intensity comprised in the haptic intensity data,
wherein a maximum and/or high intensity of the haptic intensity generated by the haptic actuator is comprised in a threshold range with respect to haptic intensity determined based on at least one of user profile data of the user, exercise attribute data of the exercise program, and the sensor data.

**FIG. 1**

**FIG. 2**

**FIG. 3**

**FIG. 4**

**FIG. 5A**

500-1

530

Motor driver circuit 532 → Motor 534

510

Memory 514

Processor 512

Communication module 516

Inertial sensor 522

First angle sensor 524

Second angle sensor 524-1

Input module 540

Sound output module 550

Haptic module 560

530-1

Motor driver circuit 532-1 → Motor 534-1

**FIG. 5B**

Electronic device ~210

Wearable device
100

Control command

212

Electronic device ~210

Wearable device
100

Control result

Sensor data

212

**FIG. 6**

210

Memory
720

Processor
710

Communication
module
730

Display module
740

Sound output
module
750

Input module
760

**FIG. 7**

Start

810
Start exercise program

820
Receive sensor data

830
Determine threshold interval of haptic
intensity of haptic feedback

840
Determine haptic intensity of haptic feedback
based on threshold interval of haptic intensity

880
Adjust threshold interval of
haptic intensity

850
No — Has haptic feedback
provision event occurred?

Yes

860
Transmit control signal to provide haptic feedback
at determined haptic intensity to wearable device

870
Determine that user
perceives haptic feedback? — No

Yes

End

**FIG. 8**

926

< 

You may receive a
recommendation for the
exercise based on
your gender.

O Male

⊙ Female          Age 50s

O Private

O Direct input

( Next )

924

You may receive a
recommendation for the
exercise based on
your gender.

⊙ Male              Age 50s

O Female

O Private

O Direct input

( Next )

User
perception
level

916

914

912

$U_3$ - - - - -

$U_2$ - - - - -

$U_1$ - - - - -

$I_{11}$ $I_{12}$ $I_{13}$

Haptic
intensity

922

You may receive a
recommendation for the
exercise based on
your gender.

O Male

⊙ Female          Age 50s

O Private

O Direct input

( Next )

**FIG. 9**

1026

Select an
exercise program

⊙ Exercise program3
(High intensity)

○ Exercise program2
(Intermediate intensity)

○ Exercise program3
(Low intensity)

Next

1024

Select an
exercise program

○ Exercise program3
(High intensity)

⊙ Exercise program2
(Intermediate intensity)

○ Exercise program1
(Low intensity)

Next

User
perception
level

1016

$P_3$
$P_2$
$P_1$

1014
1012

$I_{21} I_{22} I_{23}$

Haptic
intensity

1022

Select an
exercise program

○ Exercise program3
(High intensity)

○ Exercise program2
(Intermediate intensity)

⊙ Exercise program1
(Low intensity)

Next

**FIG. 10**

FIG. 11

<u>1210</u>

FIG. 12

FIG. 13

FIG. 14

FIG. 15

**FIG. 16A**

**FIG. 16B**

**FIG. 16C**

**FIG. 16D**

**FIG. 16E**

**FIG. 17A**

**FIG. 17B**

## INTERNATIONAL SEARCH REPORT

| | International application No. |
|---|---|
| | **PCT/KR2024/005786** |

| A. | CLASSIFICATION OF SUBJECT MATTER |
|---|---|

**G06F 3/01**(2006.01)i; **G06F 1/16**(2006.01)i; **H04W 4/80**(2018.01)i

According to International Patent Classification (IPC) or to both national classification and IPC

| B. | FIELDS SEARCHED |
|---|---|

Minimum documentation searched (classification system followed by classification symbols)

G06F 3/01(2006.01); A63F 13/285(2014.01); G01L 1/20(2006.01); G06Q 50/10(2012.01); G06Q 50/20(2012.01); H04W 4/60(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 웨어러블(wearable), 햅틱(haptic), 센서(sensor), 사용자 움직임(user motion), 사용자 프로파일(user profile), 피드백(feedback), 강도(intensity)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | KR 10-2014-0113408 A (IMMERSION CORPORATION) 24 September 2014 (2014-09-24)<br>See paragraphs [0017]-[0019], [0027], [0052], [0054] and [0060]; claims 1-4; and figure 2. | 1,3-5,9-10,12 |
| Y | | 8,15 |
| A | | 2,6-7,11,13-14 |
| Y | KR 10-2064795 B1 (ELECTRONICS AND TELECOMMUNICATIONS RESEARCH INSTITUTE) 10 January 2020 (2020-01-10)<br>See paragraph [0041]; and figure 3. | 8 |
| Y | KR 10-2022-0121194 A (INDUSTRY ACADEMIC COOPERATION FOUNDATION OF YEUNGNAM UNIVERSITY) 31 August 2022 (2022-08-31)<br>See paragraphs [0012] and [0016]. | 15 |
| A | KR 10-2018-0011458 A (INTEL CORPORATION) 01 February 2018 (2018-02-01)<br>See paragraphs [0041]-[0045]; and figure 2. | 1-15 |

| ✓ Further documents are listed in the continuation of Box C. | ✓ See patent family annex. |
|---|---|

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **01 August 2024** | **01 August 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/005786**

| C. | DOCUMENTS CONSIDERED TO BE RELEVANT | |
|---|---|---|
| Category* | Citation of document, with indication, where appropriate, of the relevant passages | Relevant to claim No. |
| A | KR 10-2018-0135804 A (IMMERSION CORPORATION) 21 December 2018 (2018-12-21)<br>See paragraphs [0008] and [0040]-[0045]; and figures 2a-2b. | 1-15 |

Form PCT/ISA/210 (second sheet) (July 2022)

## INTERNATIONAL SEARCH REPORT
### Information on patent family members

International application No.

**PCT/KR2024/005786**

| Patent document cited in search report | | | Publication date (day/month/year) | Patent family member(s) | | | Publication date (day/month/year) |
|---|---|---|---|---|---|---|---|
| KR | 10-2014-0113408 | A | 24 September 2014 | CN | 104049746 | A | 17 September 2014 |
| | | | | CN | 110658919 | A | 07 January 2020 |
| | | | | EP | 2778850 | A1 | 17 September 2014 |
| | | | | JP | 2014-209329 | A | 06 November 2014 |
| | | | | JP | 6482765 | B2 | 13 March 2019 |
| | | | | US | 2014-0267076 | A1 | 18 September 2014 |
| KR | 10-2064795 | B1 | 10 January 2020 | KR | 10-2014-0107062 | A | 04 September 2014 |
| | | | | US | 2014-0243710 | A1 | 28 August 2014 |
| KR | 10-2022-0121194 | A | 31 August 2022 | KR | 10-2679804 | B1 | 02 July 2024 |
| KR | 10-2018-0011458 | A | 01 February 2018 | CN | 107667330 | A | 06 February 2018 |
| | | | | CN | 107667330 | B | 24 August 2021 |
| | | | | EP | 3314372 | A1 | 02 May 2018 |
| | | | | JP | 2018-518754 | A | 12 July 2018 |
| | | | | JP | 6859268 | B2 | 14 April 2021 |
| | | | | US | 2016-0378186 | A1 | 29 December 2016 |
| | | | | WO | 2016-209447 | A1 | 29 December 2016 |
| KR | 10-2018-0135804 | A | 21 December 2018 | CN | 109085916 | A | 25 December 2018 |
| | | | | EP | 3416028 | A1 | 19 December 2018 |
| | | | | JP | 2019-003643 | A | 10 January 2019 |
| | | | | US | 2018-0356888 | A1 | 13 December 2018 |

Form PCT/ISA/210 (patent family annex) (July 2022)